# EUROPEAN PATENT APPLICATION

(11) **EP 1 854 784 A1**
(43) Date of publication of application: **14.11.2007**
(21) Application number: 06715252.0
(22) Date of filing: 28.02.2006
(51) Int. Cl.: C07D 207/337, A61K 31/341, A61K 31/381, A61K 31/40, A61K 31/415, A61K 31/443, A61K 31/4436, A61K 31/4439, A61P 3/04, A61P 3/06, A61P 3/10, A61P 43/00, C07D 231/12, C07D 307/54, C07D 333/24, C07D 401/04, C07D 405/04, C07D 409/04

(54) **ACTIVATOR FOR PEROXISOME PROLIFERATOR ACTIVATING RECEPTOR**

(30) Priority: 28.02.2005 JP 2005052762
(71) Applicant: Nippon Chemiphar Co., Ltd., Chiyoda-ku Tokyo 1010032 (JP)
(72) Inventor: SAKUMA, Shogo, Saitama, 3420041 (JP); MOCHIDUKI, Nobutaka, Chiba, 2701166 (JP); TAKAHASHI, Rie, Misato-shi, Saitama, 3410005 (JP); HIRAI, Toshitake, Chiba, 2780036 (JP); YAMAKAWA, Tomio, Chiba, 2770884 (JP); MASUI, Seiichiro, Saitama 3620072 (JP)
(74) Representative: Hermann, Bettina Celia
(86) International application number: PCT/JP2006/304193
(87) International publication number: WO 2006/090920

(57) **Abstract**

A compound or its salt having the following formula (II) is used as an activator for PPAR δ: 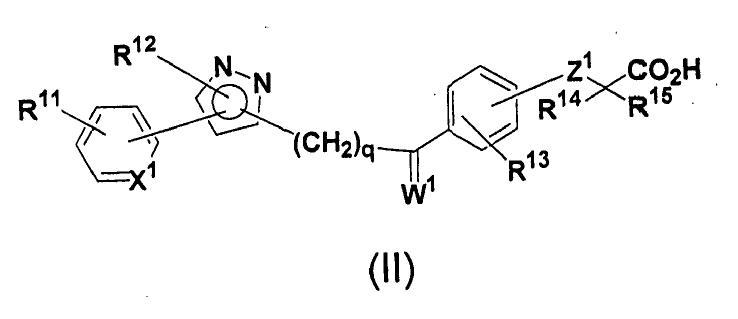 [in which each of R¹¹ and R¹³ is a hydrogen atom, an alkyl group having 1 to 8 carbon atoms, an alkoxy group having 1 to 8 carbon atoms, an alkyl group having 1 to 8 carbon atoms and a halogen atom substituent, etc.; R¹² is a hydrogen atom, an alkyl group having 1 to 8 carbon atoms, an alkoxy group having 1 to 8 carbon atoms, an alkyl group having 1 to 8 carbon atoms and a halogen atom substituent, etc.; each of R¹⁴ and R¹⁵ is a hydrogen atom, an alkyl group having 1 to 8 carbon atoms, or an alkyl group having 1 to 8 carbon atoms and a halogen atom substituent; X¹ is CH or N; Z¹ is an oxygen atom or a sulfur atom; W¹ is an oxygen atom or CH₂; and q is an integer of 2 to 4].

## Description

### [FIELD OF THE INVENTION]

The present invention relates to an activator for peroxisome proliferator activated receptor (PPAR) δ.

### [BACKGROUND OF THE INVENTION]

As for the peroxisome proliferator activated receptor (PPAR), it is known that there are three subtypes such as PPARα, PPARγ and PPARδ. --- Proc. Natl. Acad. Sci. USA, 91, p7335-7359, 1994.

Until now, a transcription-activating function, a glycemic index-depressing function, and a lipid metabolism-improving function have been reported on each subtype of PPAR.

For instance, WO 97/28115 (Patent Publication 1) describes use of L-165041 (Merck) as a diabetes treatment medicine and an anti-obesity medicine; WO 99/04815 (Patent Publication 2) describes that YM-16638 (Yamanouchi) has a serum cholesterol-depressing function and an LDL cholesterol-depressing function; and WO 2004/7439 (Patent Publication 3) describes use of biaryl derivatives as medicines for enhancing a blood HDL. In addition, a large number of patent applications, namely, WO 01/40207 (Patent Publication 4: GW-590735, GSK), WO 2004/63166 (Patent Publication 5: pyrazole derivatives, Lilly), WO 02/092590 (Patent Publication 6: thiophene derivatives, GSK), WO 03/099793 (Patent Publication 7: azole derivatives, Takeda), have been filed.

WO 01/603 (Patent Publication 8) describes that GW-501516 (GSK) having the following formula: 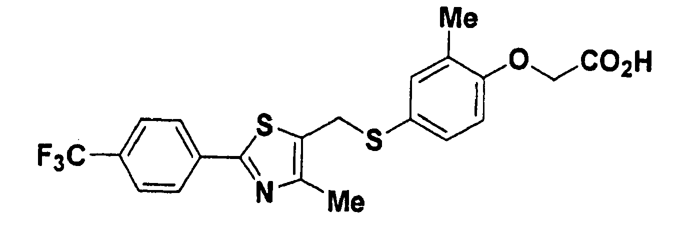 is being studied as a lipid metabolism-improving medicine.

The present inventors have filed patent applications (WO 02/14291 (Patent Publication 9), WO 03/16291 (Patent Publication 10), etc.) for compounds having a thiazole ring and the like which have a transcription-activating function.

There are clear structural differences between the above-illustrated GW-501516 and the compounds of the present invention which are represented by the below-illustrated formula (I), (II), or (III). Further, the latter compounds are not clearly described in the aforementioned patent publications.

### [DISCLOSURE OF THE INVENTION]

The invention has an object to provide compounds having the following formula (I), (II) or (III), which have an activating function for peroxisome proliferator-activated receptor.

In one aspect, the invention resides in compounds having the following formula (I) or salts thereof: 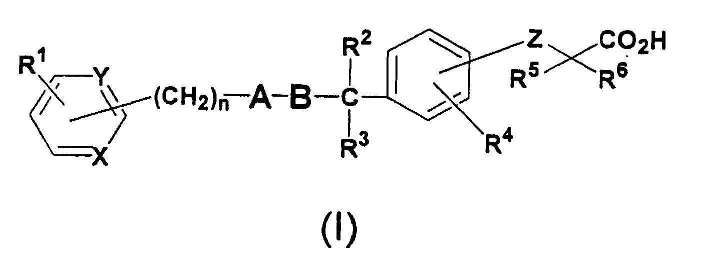 in which
R¹ and R⁴ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;
R² represents a hydrogen atom;
R³ represents an alkyl group having 1 to 8 carbon atoms, or R³ is combined with R² to represent =0 or =C(R⁷)(R⁸) in which R⁷ and R⁸ are the same or different and each represents a hydrogen atom or an alkyl group having 1 to 8 carbon atoms;
R⁵ and R⁶ are the same or different and each represents a hydrogen atom, an alkyl group having 1 to 8 carbon atoms, or an alkyl group having 1 to 8 carbon atoms and a halogen atom substituent;
X and Y are the same or different and each represents CH or N;
Z represents an oxygen atom or a sulfur atom;
A represents a 5-membered heterocyclic group selected from the group consisting of pyrazole, thiophene, furan and pyrrole which optionally has an alkyl substituent having 1 to 8 carbon atoms which has a substituent selected from the group consisting of an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group which has 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and 5- or 6-membered heterocyclic group;
B represents an alkylene chain having 1 to 8 carbon atoms which optionally has a substituent selected from the group consisting of an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, and an alkoxy group having 1 to 8 carbon atoms and a halogen atom substituent, the alkylene group optionally having a double bond in the case that the alkylene group has 2 to 6 carbon atoms;
and
n is an integer of 0 to 5.

In another aspect, the invention resides in compounds having the following formula (II) or salts thereof: 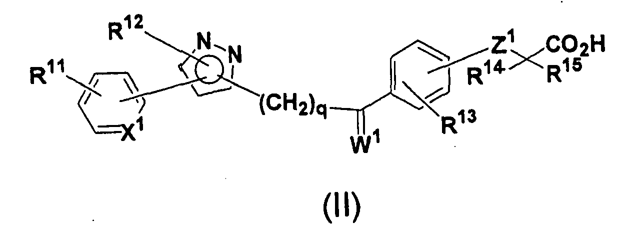 in which
R¹¹ and R¹³ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;
R¹² represents a hydrogen atom, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent;

R¹⁴ and R¹⁵ are the same or different and each represents a hydrogen atom, an alkyl group having 1 to 8 carbon atoms, or an alkyl group having 1 to 8 carbon atoms and a halogen atom substituent;
X¹ represents CH or N;
Z¹ represents an oxygen atom or a sulfur atom;
W¹ represents an oxygen atom or CH₂;
and
q is an integer of 2 to 4.

In still another aspect, the invention resides in compounds having the following formula (III) or salts thereof: 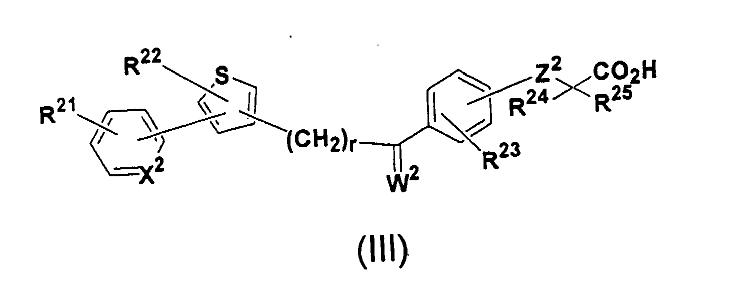 in which
R²¹ and R²³ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;

R²² represents a hydrogen atom, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent;

R²⁴ and R²⁵ are the same or different and each represents a hydrogen atom, an alkyl group having 1 to 8 carbon atoms, or an alkyl group having 1 to 8 carbon atoms and a halogen atom substituent;
X² represents CH or N;
Z² represents an oxygen atom or a sulfur atom;
W² represents an oxygen atom or CH₂;
and
r is an integer of 2 to 4.

In still another aspect, the invention resides in an activator for peroxisome proliferator activated receptor δ containing a compound of the formulas (I), (II) or (III) as an effective component.

### [PREFERRED EMBODIMENTS OF THE INVENTION]

The invention is described below in detail.

Regarding the formula (I), examples of the alkyl groups having 1 to 8 carbon atoms which can be R¹, R³, R⁴, R⁵, R⁶, R⁷, the substituent of the 5-membered heterocyclic group for A, or the substituent of the alkylene chain having 2 to 6 carbon atoms for B include methyl, ethyl, propyl, isopropyl, butyl, i-butyl, t-butyl, pentyl or hexyl.

Examples of the alkenyl groups having 2 to 8 carbon atoms which can be R¹, R⁴, the substituent of the 5-membered heterocyclic group for A, or the substituent of the alkylene chain having 2 to 6 carbon atoms for B include vinyl and allyl.

Examples of the alkynyl groups having 2 to 8 carbon atoms which can be R¹, R⁴, the substituent of the 5-membered heterocyclic group for A, or the substituent of the alkylene chain having 2 to 6 carbon atoms for B include propargyl.

Examples of the 3- to 7-membered cycloalkyl groups which can be the substituent of the 5-membered heterocyclic group for A, or the substituent of the alkylene chain having 2 to 6 carbon atoms for B include cyclopropyl, cyclopentyl and cyclohexyl.

Examples of the alkoxy groups having 1 to 8 carbon atoms which can be R¹, R⁴, the substituent of the 5-membered heterocyclic group for A, or the substituent of the alkylene chain having 2 to 6 carbon atoms for B include methoxy, ethoxy, propoxy, isopropoxy, butoxy, i-butoxy, t-butoxy, pentyloxy and hexyloxy.

Examples of the halogen atoms which can be R¹, R⁴, or the substituent of the alkylene chain having 2 to 6 carbon atoms for B include fluorine, chlorine, and bromine.

Examples of the alkyl groups having 1 to 8 carbon atoms and a halogen atom substituent which can be R¹, R⁴, R⁵, R⁶, the substituent of the 5-membered heterocyclic group for A, or the substituent of the alkylene chain having 2 to 6 carbon atoms for B include methyl, ethyl, propyl, isopropyl, butyl and t-butyl which have substituents such as 1 to 3 fluorine, chlorine or bromine atoms. Preferred are trifluoromethyl, chloromethyl, 2-chloroethyl, 2-bromoethyl, and 2-fluoroethyl.

Examples of the alkoxy groups having 1 to 8 carbon atoms and a halogen atom substituent which can be R¹, R⁴, the substituent of the 5-membered heterocyclic group for A, or the substituent of the alkylene chain having 2 to 6 carbon atoms for B include methoxy, ethoxy, propoxy, iso-propyloxy, butyloxy and t-butyloxy which have substituents such as 1 to 3 fluorine, chlorine or bromine atoms. Preferred are trifluoromethyloxy, chloromethyloxy, 2-chloroethyloxy, 2-bromoethyloxy, and 2-fluoroethyloxy.

Examples of the acyl groups having 2 to 8 carbon atoms which can be R¹ or R⁴, include acetyl and propionyl.

Examples of the aryl groups having 6 to 10 carbon atoms which can be R¹, R⁴, or the substituent of the 5-membered heterocyclic group for A, include phenyl.

Examples of the 5- or 6-membered heterocyclic groups which can be R¹, R⁴, or the substituent of the 5-membered heterocyclic group for A, include pyridyl.

Examples of the alkyl groups having 1 to 8 carbon atoms and a 3- to 7-cycloalkyl group substituent which can be the substituent of the 5-membered heterocyclic group for A, include methyl, ethyl, propyl, isopropyl, butyl, i-butyl, t-butyl, pentyl and hexyl which have cyclopropyl, cyclopentyl, or cyclophexyl substituent.

Examples of the aralkyl groups (which have an aryl moiety of 6 to 10 carbon atoms and an alkylene moiety of 1 to 8 carbon atoms) which can be the substituent of the 5-membered heterocyclic group for A, include benzyl and phenethyl.

Examples of the alkyl groups having 1 to 8 carbon atoms and a 5- or 6-membered heterocyclic group which can be the substituent of the 5-membered heterocyclic group for A, include methyl, ethyl, propyl, isopropyl, butyl, i-butyl, t-butyl, pentyl and hexyl which have a pyridyl substituent.

Examples of the alkyl groups having 1 to 8 carbon atoms, alkenyl groups having 2 to 8 carbon atoms, alkynyl groups having 2 to 8 carbon atoms, alkoxy groups having 1 to 8 carbon atoms, halogen atoms, alkyl groups having 1 to 8 carbon atoms and a halogen atom substituent, alkoxy groups having 1 to 8 carbon atoms and a halogen atom substituent, acyl groups having 2 to 8 carbon atoms, aryl groups having 6 to 10 carbon atoms, and 5- or 6-membered heterocyclic groups which can be R¹¹ or R¹³ of the formula (II) or R²¹ or R²³ of the formula (III) are those described hereinabove for R¹ and R⁴ of the formula (I).

Examples of the alkyl groups having 1 to 8 carbon atoms, 3- to 7-membered cycloalkyl groups, alkenyl groups having 2 to 8 carbon atoms, alkynyl groups having 2 to 8 carbon atoms, alkoxy groups having 1 to 8 carbon atoms, alkyl groups having 1 to 8 carbon atoms and a 3- to 7-membered cycloalkyl group substituent, alkyl groups having 1 to 8 carbon atoms and a halogen atom substituent, alkoxy groups having 1 to 8 carbon atoms and a halogen atom substituent, aryl groups having 6 to 10 carbon atoms, 5- or 6-membered heterocyclic groups, aralkyl groups having an aryl moiety of 6 to 10 carbon atoms and an alkylene moiety of 1 to 8 carbon atoms, and alkyl groups having 1 to 8 carbon atoms and a 5- or 6-membered heterocyclic substituent which can be R¹² of the formula (II) or R²² of the formula (III) include those described hereinabove for the substituent of the 5-membered heterocyclic group for A of the formula (I).

Examples of the alkyl groups having 1 to 8 carbon atoms and alkyl groups having 1 to 8 carbon atoms and a halogen atom substituent which can be R¹⁴ or R¹⁵ of the formula (II) or R²⁴ or R²⁵ of the formula (III) include those described hereinabove for R⁵ and R⁶ of the formula (I).
R¹ in the formula (I), R¹¹ in the formula (II), and R²¹ in the formula (III) can be attached to the benzene ring or the like in a single or plural number (1 to 3). If each of R¹, R¹¹ and R²¹ is present in a plural number, the plural groups can be the same or different.

R⁴ in the formula (I), R¹³ in the formula (II), and _{R}²³ in the formula (III) can be attached to the benzene ring or the like in a single or plural number (1 to 3). If each of R⁴, R¹³ and R²³ is present in a plural number, the plural groups can be the same or different.

The substituent group of the 5-membered heterocyclic group for A in the formula (I), R¹² in the formula (II), and R²² in the formula (III) can be attached to the heterocyclic ring in a single or plural number (1 or 2). If each of the substituent group of the 5-membered heterocyclic group for A, R¹² and R²² is present in plural number, the plural groups can be the same or different.

The preferred compounds according to the invention are described below.
(1) Compounds of the formula (I) in which A is pyrazole, and salts thereof.
(2) Compounds of (1) above in which -(CH₂)ₙ- is attached the pyrazole at 1-position thereof, and salts thereof.
(3) Compounds of (1) above in which -(CH₂)ₙ- is attached the pyrazole at 3-position thereof, and salts thereof.
(4) Compounds of (2) or (3) above in which -B- is attached the pyrazole at 4-or 5-position thereof, and salts thereof.
(5) Compounds of the formula (I) in which A is thiophene, furan or pyrrole, and salts thereof.
(6) Compounds of (5) above in which -(CH₂)ₙ- is attached the 5-membered heterocyclic group at 2-position thereof, and salts thereof.
(7) Compounds of the formula (I) in which A is thiophene, and salts thereof.
(8) Compounds of (7) above in which -(CH₂)ₙ- is attached the thiophene at 2-position thereof, and salts thereof.
(9) Compounds of the formula (I) or one of (1) to (8) above in which n is 0, and salts thereof.
(10) Compounds of the formula (I) or one of (1) to (9) above in which each of X and Y is CH, and salts thereof.
(11) Compounds of the formula (I) or one of (1) to (10) above in which R² is combined with R³ to represent =O, and salts thereof.
(12) Compounds of the formula (I) or one of (1) to (11) above in which B represents an alkylene chain having 2 to 4 carbon atoms which optionally has a substituent selected from the group consisting of an alkyl group having 1 to 8 carbon atoms and an alkyl group having 1 to 8 carbon atoms and a halogen atom substituent, and salts thereof.
(13) Compounds of the formula (I) or one of (1) to (12) above in which B is an ethylene chain, and salts thereof.
(14) Compounds of the formula (I) or one of (1) to (13) above in which R¹ and R⁴ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent, and salts thereof.
(15) Compounds of the formula (I) or one of (1) to (14) above in which R⁵ and R⁶ are the same or different and each represents a hydrogen atom or an alkyl group having 1 to 8 carbon atoms, and salts thereof.
(16) Compounds of the formula (I) or one of (1) to (15) above in which the substituent optionally attached to the heterocyclic group for A is an alkyl group having 1 to 8 carbon atoms or an alkyl group having 1 to 8 carbon atoms and a halogen atom substituent, and salts thereof.
(17) Compounds of the formula (II) in which X¹ is CH, and salts thereof.
(18) Compounds of the formula (II) in which R¹¹-phenyl or R¹¹-pyridyl is attached to the pyrazole at 1-position, and salts thereof.
(19) Compounds of the formula (II) in which R¹¹-phenyl or R¹¹-pyridyl is attached to the pyrazole at 3-position, and salts thereof.
(20) Compounds of the formula (II) or one of (17) to (19) above in which -(CH₂)_{q}C(=W¹)- is attached to the pyrazole at 4- or 5-position, and salts thereof.
(21) Compounds of the formula (II) or one of (17) to (20) above in which W¹ is an oxygen atom, and salts thereof.
(22) Compounds of the formula (II) or one of (17) to (21) above in which R¹¹ and R¹³ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent, and salts thereof.
(23) Compounds of the formula (II) or one of (17) to (21) above in which R¹¹ and R¹³ are the same or different and each represents an alkyl group having 1 to 8 carbon atoms or an alkyl group having 1 to 8 carbon atoms and a halogen atom substituent, and salts thereof.
(24) Compounds of the formula (II) or one of (17) to (23) above in which R¹⁴ and R¹⁵ are the same or different and each represents a hydrogen atom or an alkyl group having 1 to 8 carbon atoms, and salts thereof.
(25) Compounds of the formula (II) or one of (17) to (24) above in which R¹² represents a hydrogen atom, an alkyl group having 1 to 8 carbon atoms, an alkoxy group having 1 to 8 carbon atoms, an alkyl group having 1 to 8 carbon atoms and a halogen atom substituent, or an alkoxy group having 1 to 8 carbon atoms and a halogen atom substituent, and salts thereof.
(26) Compounds of the formula (II) or one of (17) to (24) above in which R¹² represents an alkyl group having 1 to 8 carbon atoms, or an alkyl group having 1 to 8 carbon atoms and a halogen atom substituent, and salts thereof.
(27) Compounds of the formula (II) or one of (17) to (26) above in which q is 2, and salts thereof.
(28) Compounds of the formula (III) in which X² is CH, and salts thereof.
(29) Compounds of the formula (III) in which R²¹-phenyl or R²¹-pyridyl is attached to the thiophene at 2-position, and salts thereof.
(30) Compounds of the formula (III) or (28) or (29) above in which W² is an oxygen atom, and salts thereof.
(31) Compounds of the formula (III) or one of (28) to (30) above in which R²¹ and R²³ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent, and salts thereof.
(32) Compounds of the formula (III) or one of (28) to (30) above in which R²¹ and R²³ are the same or different and each represents an alkyl group having 1 to 8 carbon atoms or an alkyl group having 1 to 8 carbon atoms and a halogen atom substituent, and salts thereof.
(33) Compounds of the formula (III) or one of (28) to (32) above in which R²⁴ and R²⁵ are the same or different and each represents a hydrogen atom or an alkyl group having 1 to 8 carbon atoms, and salts thereof.
(34) Compounds of the formula (III) or one of (28) to (33) above in which R²² represents a hydrogen atom, an alkyl group having 1 to 8 carbon atoms, an alkoxy group having 1 to 8 carbon atoms, an alkyl group having 1 to 8 carbon atoms and a halogen atom substituent, or an alkoxy group having 1 to 8 carbon atoms and a halogen atom substituent, and salts thereof.
(35) Compounds of the formula (III) or one of (28) to (33) above in which R²² represents an alkyl group having 1 to 8 carbon atoms, or an alkyl group having 1 to 8 carbon atoms and a halogen atom substituent, and salts thereof.
(36) Compounds of the formula (III) or one of (28) to (33) above in which r is 2, and salts thereof.

The compounds of the formulas (I), (II) and (III) can be pharmacologically acceptable salts such as alkali metal salts, for example, sodium salts, potassium salts, or lithium salts.

The compounds of the invention can be present in the optically active forms, and in the form of optical isomers such as compounds of a racemic form or geometric isomers such as compounds of a cis- or trans form.

The schemes for synthesis of the compounds of the invention having the formula (I) are illustrated below.

### [Synthesis Process 1: in the case that Z is 0, and R² and R³ are combined to form =O]

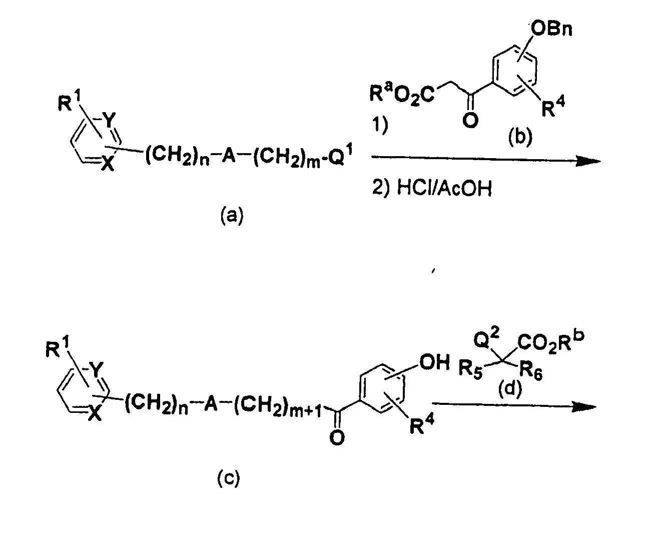

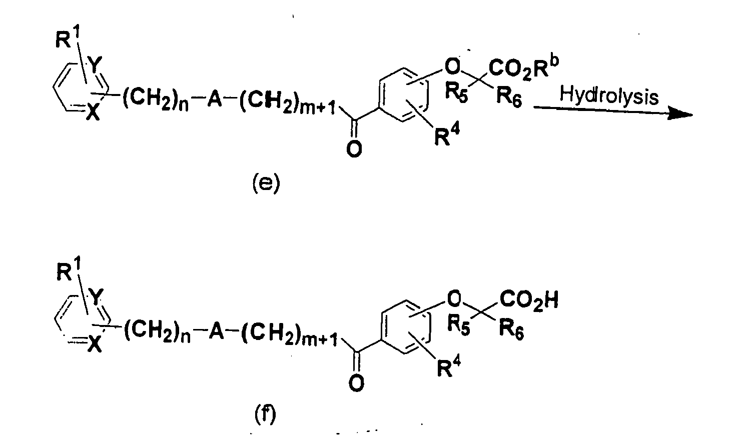

In the formula, each of Q¹ and Q² is a halogen atom such as chlorine or bromine; each of R^{a} and R^{b} is a lower alkyl group such as ethyl; Bn is benzyl; m is an integer of 1 to 5; and each of R¹, R⁴, R⁵, R⁶, X, Y, A and n has the aforementioned meaning.

The phenol compound represented by the formula (c) can be prepared by the steps of reaction of a compound of the formula (a) with a compound of the formula (b) in an inert solvent such as THF in the presence of a base such as sodium hydride; decarboxylation using hydrochloric acid-acetic acid, and removal of the benzyl group.

Subsequently, the phenol compound of the formula (c) and a compound of the formula (d) are reacted in an inert solvent such as 2-butanone in the presence of a base such as potassium carbonate, to give an ester compound represented by the formula (e).

The ester compound of the formula (e) is then subjected to hydrolysis in the presence of a base such as sodium hydroxide, potassium carbonate, or lithium hydroxide, to give the compound of the invention having the formula (f).

### [Synthesis Process 2: in the case that Z is O, and R² and R³ are combined to form =CH₂]

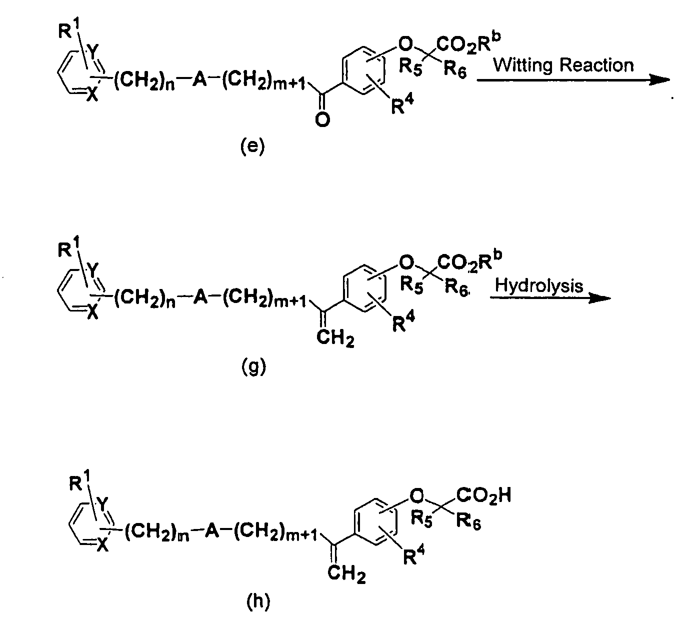

In the formula, each of R^{b}, R¹, R⁴, R⁵, R⁶, X, Y, A, m and n has the aforementioned meaning.

The exomethylene compound represented by the formula (g) can be prepared by reacting a Wittig reagent (which can be obtained by reacting methyltriphenylphosphonyl bromide with a strong base such as sodium amide) and the ester compound of the formula (e) illustrated in Synthesis Process 1.

Thus obtained exomethylene compound of the formula (g) is subjected to hydrolysis in the presence of a base such as sodium hydroxide or potassium carbonate, to give the compound of the invention having the formula (h).

### [Synthesis Process 3: in the case that Z is O, R² is H, and R³ is -CH₃]

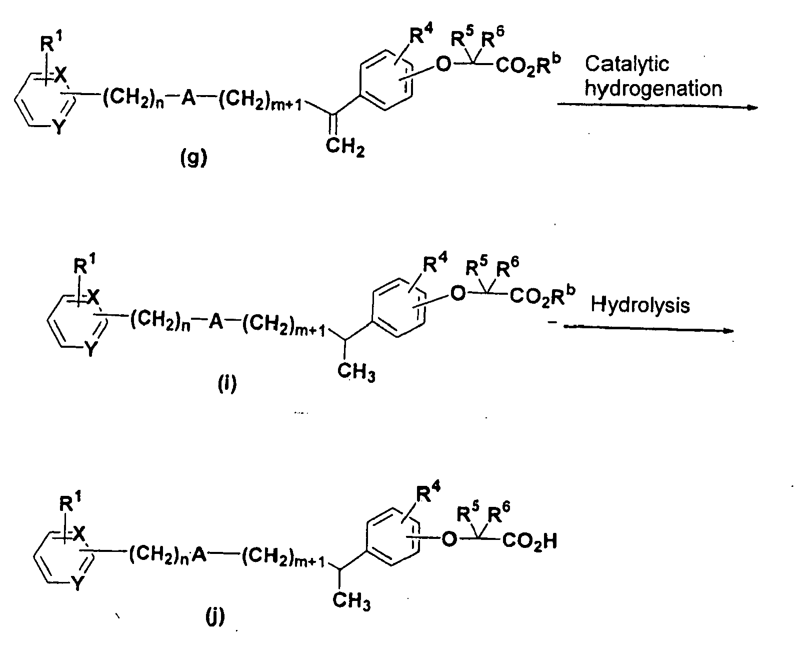

In the formula, each of R^{b}, R¹, R⁴, R⁵, R⁶, X, Y, A, m and n has the aforementioned meaning.

The ester compound represented by the formula (i) can be obtained by subjecting the exomethylene compound of the formula (g) illustrated in Synthesis Process 2 to catalytic hydrogenation using palladium/carbon.

Thus obtained ester compound of the formula (i) is then subjected to hydrolysis in the presence of a base such as sodium hydroxide or potassium carbonate, to give the compound of the invention having the formula (j).

### [Synthesis Process 4: in the case that Z is O, R² and R³ are combined to form =0]

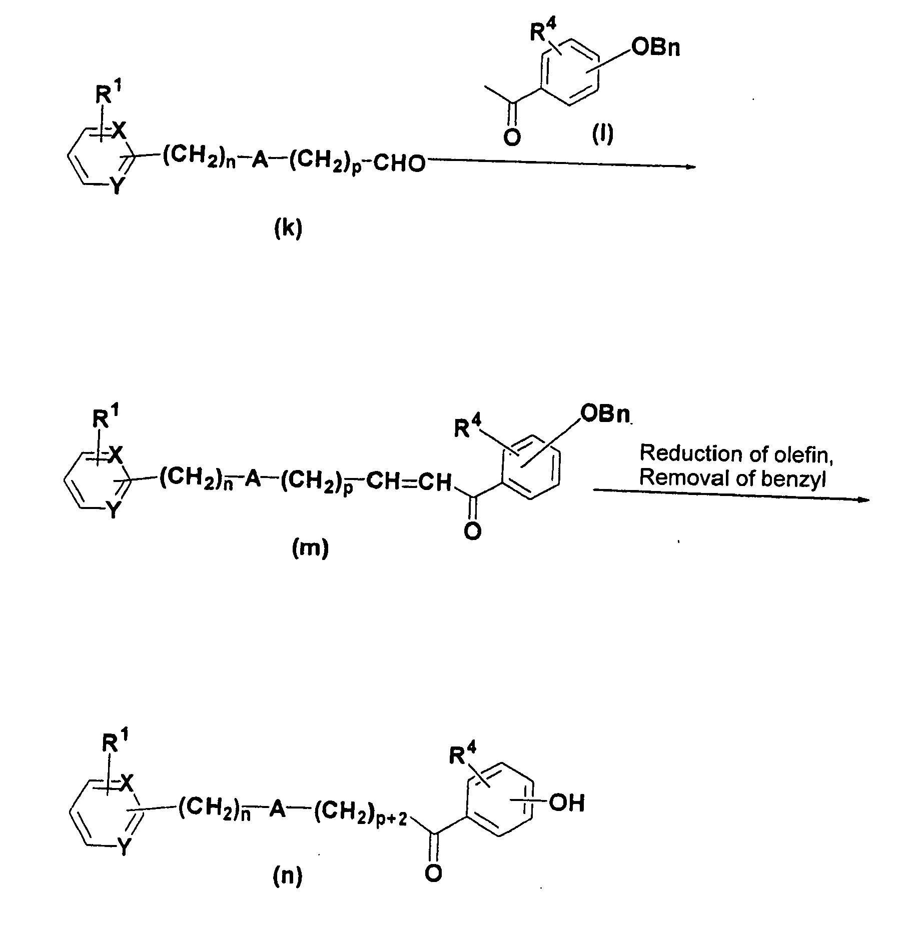

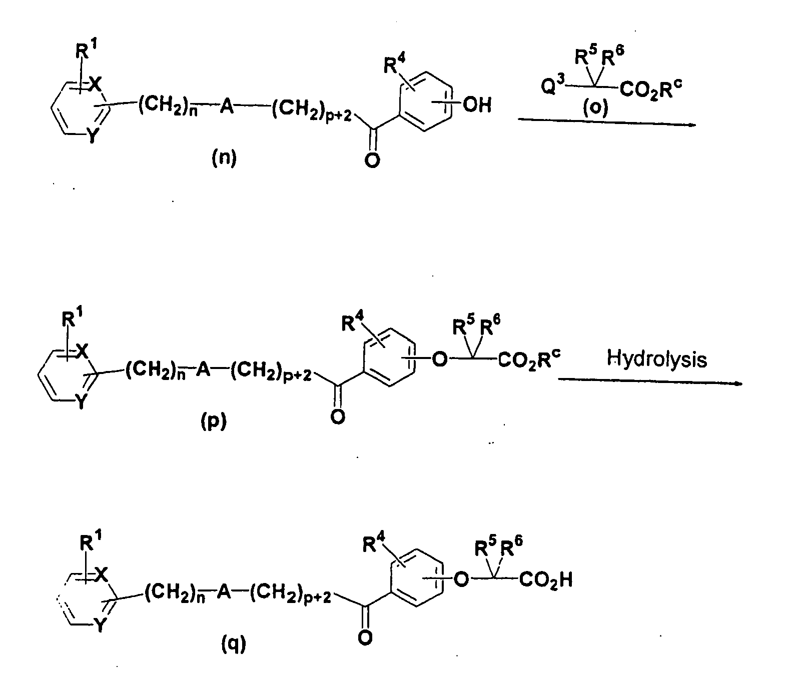

In the formula, Q³ is a halogen atom such as chlorine or bromine; R^{c} is a lower alkyl group such as ethyl; p is an integer of 0 to 4; and each of R¹, R⁴, R⁵, R⁶, X, Y, Bn, A and n has the aforementioned meaning.

The vinyl compound represented by the formula (m) can be obtained by subjecting an aldehyde compound of the formula (k) and an acetophenone compound of the formula (1) to an aldol condensation reaction in the presence of a base. The phenol compound represented by the formula (n) can be obtained by subjecting the vinyl compound of the formula (m) to a reaction for reducing the olefinic moiety and subsequently to a benzyl group-removing reaction.

Subsequently, the phenol compound of the formula (n) and a compound of the formula (o) are reacted, to give an ester compound of the formula (p).

Thus obtained ester compound of the formula (p) is then subjected to hydrolysis in the presence of a base such as sodium hydroxide or potassium carbonate, to give the compound of the invention having the formula (q).

### [Synthesis Process 5: in the case that Z is O, R² and R³ are combined to form =CH₂]

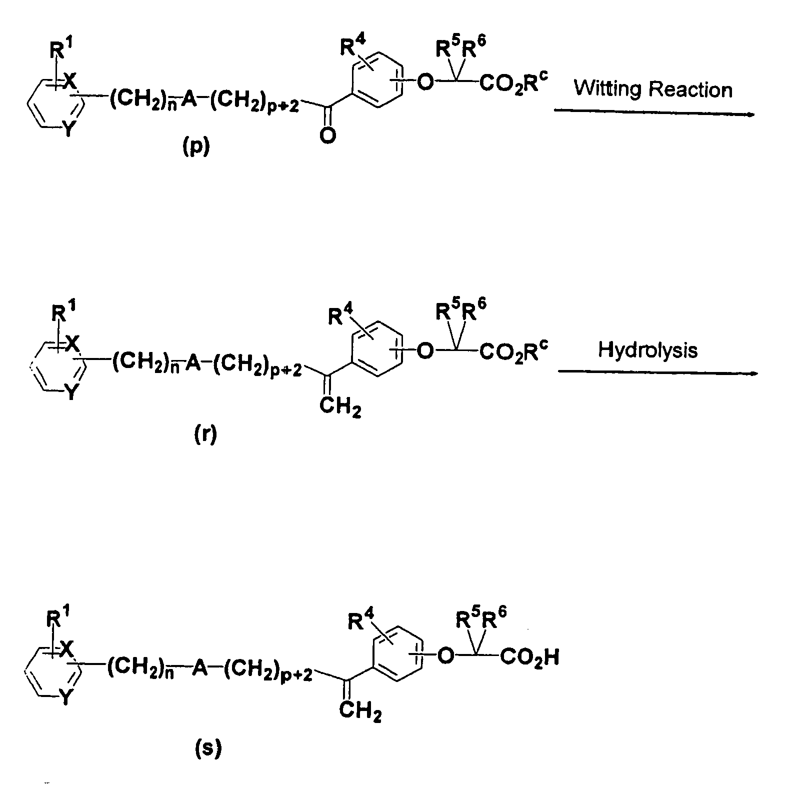

In the formula, each of R^{c}, R¹, R⁴, R⁵, R⁶, X, Y, A, p and n has the aforementioned meaning.

The exomethylene compound represented by the formula (r) can be obtained by reacting the ester compound of the formula (p) with a Wittig reagent.

Thus obtained exomethylene compound of the formula (r) is then subjected to hydrolysis in the presence of a base such as sodium hydroxide or potassium carbonate, to give the compound of the invention having the formula (s).

Further, the compounds of the invention (1) can be prepared by the below-mentioned working examples and with reference to the aforementioned patent publications and other publications.

Examples of the compounds of the invention are set forth in the following tables.
(1) Compounds represented by the following formula: 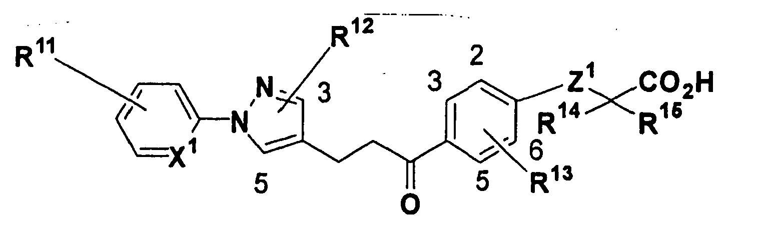 in which each of R¹¹ R¹² R¹³, R¹⁴, R¹⁵, X¹ and Z¹ is that set forth in Tables 1 and 2.

**Table 1**

| X¹ | Z¹ | R¹¹ | R¹² | R¹³ | R¹⁴/R¹⁵ |
|---|---|---|---|---|---|
| CH | O | 4-CF₃ | 3-iPr | 2-Me | Me/Me |
| CH | O | 4-CF₃ | 3-iPr | 2-Me | H/H |
| N | O | 5-Me | 3-iPr | 2-Me | Me/Me |
| N | O | 5-Me | 3-iPr | 2-Me | H/H |
| CH | O | 4-CF₃ | 3-hexyl(Hex) | 2-Me | Me/Me |
| CH | O | 4-CF₃ | 3-Hex | 2-Me | H/H |
| CH | S | 4-Me | 3-Hex | 2-Me | Me/Me |
| CH | S | 4-Me | 3-Hex | 2-Me | H/H |
| CH | O | 4-CF₃ | 3-secBu, 5-Me | 3-Me | Me/Me |
| CH | O | 4-CF₃ | 3-secBu, 5-Me | 3-Me | H/H |
| CH | O | 4-CF₃ | 3-cyclopropyl | 2,6-Me | Me/Me |
| CH | O | 4-CF₃ | 3-cyclopropyl | 2,6-Me | H/H |
| N | O | 5-Me | 3-iPr | 2-allyl | Me/Me |
| N | O | 5-Me | 3-iPr | 2-allyl | H/H |

**Table 2**

| X¹ | Z¹ | R¹¹ | R¹² | R¹³ | R¹⁴/R¹⁵ |
|---|---|---|---|---|---|
| CH | S | 4-OMe | 3-iPr, 5-Me | 2-CF₃ | Me/Me |
| CH | S | 4-OMe | 3-iPr, 5-Me | 2-CF₃ | H/H |
| CH | O | 4-CF₃ | 3-CH₂OBu | 2-Me | Me/Me |
| CH | O | 4-CF₃ | 3-CH₂OBu | 2-Me | H/H |
| CH | O | 4-OCF₃ | 3-(CH₂)₅OMe | 2-Me | Me/Me |
| CH | O | 4-OCF₃ | 3-(CH₂)₅OMe | 2-Me | H/H |
| CH | S | 4-CF₃ | 3-Hex, 5-Me | 2-F | Me/Me |
| CH | S | 4-CF₃ | 3-Hex, 5-Me | 2-F | H/H |
| CH | O | 4-CF₃ | 3-O(CH₂)₅OMe | 2,5-Me | Me/Me |
| CH | O | 4-CF₃ | 3-O(CH₂)₅OMe | 2, 5-Me | H/H |
| N | O | 5-Me | 3-OiPr | 2-Me | Me/Me |
| N | O | 5-Me | 3-OiPr | 2-Me | H/H |

(2) Compounds represented by the following formula: 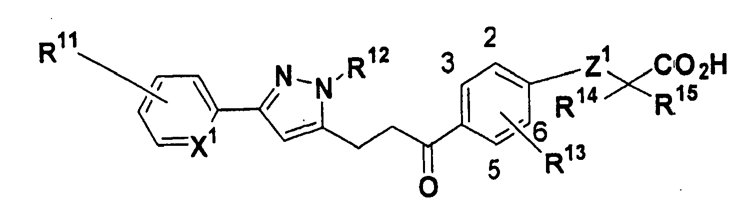 in which each of R¹¹, R¹², R¹³, R¹⁴, R¹⁵, X¹ and Z¹ is that set forth in Tables 3 and 4.

**Table 3**

| X¹ | Z¹ | R¹¹ | R¹² | R¹³ | R¹⁴/R¹⁵ |
|---|---|---|---|---|---|
| CH | O | 4-CF₃ | 3-iPr | 2-Me | Me/Me |
| CH | O | 4-CF₃ | 3-iPr | 2-Me | H/H |
| CH | O | 4-CF₃ | 3-iPr | 2-Me | Me/H |
| CH | O | 4-CF₃ | Hex | 2-Me | Me/Me |
| CH | O | 4-CF₃ | Hex | 2-Me | H/H |
| CH | O | 4-Me | Hex | 2-Me | Me/Me |
| CH | O | 4-Me | Hex | 2-Me | H/H |
| CH | S | 4-Me | Hex | 2-Me | Me/Me |
| CH | S | 4-Me | Hex | 2-Me | H/H |
| CH | O | 4-CF₃ | secBu | 2,3-Me | Me/Me |
| CH | O | 4-CF₃ | secBu | 2,3-Me | H/H |
| CH | O | 4-CF₃ | cyclopropyl | 2,6-Me | Me/Me |
| CH | O | 4-CF₃ | cyclopropyl | 2,6-Me | H/H |
| N | O | 5-Me | iPr | 2-allyl | Me/Me |
| CH | O | 4-CF₃ | cyclopropylmethyl | 2-Me | H/H |
| CH | O | 4-CF₃ | cyclopropylmethyl | 2-Me | Me/Me |
| CH | O | 4-CF₃ | benzyl | 2-Me | Me/Me |
| CH | O | 4-CF₃ | benzyl | 2-Me | H/H |

**Table 4**

| X¹ | Z¹ | R¹¹ | R¹² | R¹³ | R¹⁴/R¹⁵ |
|---|---|---|---|---|---|
| N | O | 5-Me | iPr | 2-allyl | H/H |
| CH | O | 4-OCF₃ | iPr | 2-CF₃ | Me/Et |
| CH | O | 4-OCF₃ | iPr | 2-CF₃ | H/H |
| CH | S | 4-CF₃ | (CH₂)₂OBu | 2-Me | Me/Me |
| CH | S | 4-CF₃ | (CH₂)₂OBu | 2-Me | H/H |
| CH | O | 4-CF₃ | (CH₂)₅OMe | 2-OMe | Me/Me |
| CH | O | 4-CF₃ | (CH₂)₅OMe | 2-OMe | H/H |
| CH | S | 3,4-Me | Hex | 2-F | Me/Me |
| CH | S | 3,4-Me | Hex | 2-F | H/H |
| N | O | 5-CF₃ | iPr | 2,5-Me | Me/Me |
| N | O | 5-CF₃ | iPr | 2,5-Me | H/H |
| N | O | 5-Me | Hex | H | Me/Me |
| N | O | 5-Me | Hex | H | H/H |

(3) Compounds represented by the following formula: 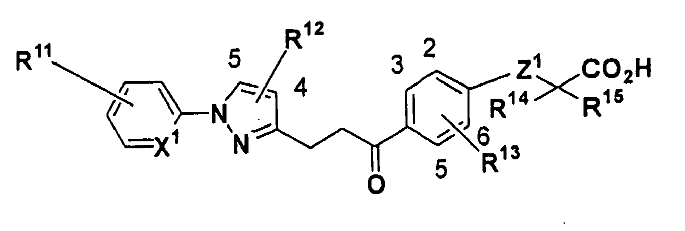 in which each of R¹¹, R¹², R¹³, R¹⁴, R¹⁵ X¹ and Z¹ is that set forth in Tables 5 and 6.

**Table 5**

| x¹ | Z¹ | R¹¹ | R¹² | R¹³ | R¹⁴/R¹⁵ |
|---|---|---|---|---|---|
| CH | O | 4-CF₃ | 4-iPr | 2-Me | Me/Me |
| CH | O | 4-CF₃ | 4-iPr | 2-Me | H/H |
| N | S | 5-CF₃ | 4-iPr | 2-Me | Me/Me |
| N | S | 5-CF₃ | 4-iPr | 2-Me | H/H |
| CH | O | 4-CF₃ | 4-Hex | 2-Me | Me/Me |
| CH | O | 4-CF₃ | 4-Hex | 2-Me | H/H |
| CH | O | 4-Me | 4-Hex | 3-Me | Me/Me |
| CH | O | 4-Me | 4-Hex | 3-Me | H/H |
| CH | O | 4-CF₃ | 4-secBu, 5-Me | 2,5-Me | Me/Me |
| CH | O | 4-CF₃ | 4-secBu, 5-Me | 2,5-Me | H/H |
| CH | O | 4-CF₃ | 4-cyclopropyl | 2,6-Me | Me/Me |
| CH | O | 4-CF₃ | 4-cyclopropyl | 2,6-Me | H/H |
| N | O | 5-Me | 5-iPr | 2-allyl | Me/Me |
| N | O | 5-Me | 5-iPr | 2-allyl | H/H |

**Table 6**

| X¹ | Z¹ | R¹¹ | R¹² | R¹³ | R¹⁴/R¹⁵ |
|---|---|---|---|---|---|
| CH | O | 4-OMe | 4-iPr, 5-Me | 2-CF₃ | Me/Me |
| CH | O | 4-OMe | 4-iPr, 5-Me | 2-CF₃ | H/H |
| CH | S | 3,4-Me | 4-CH₂OBu | 2-Me | Me/Me |
| CH | S | 3,4-Me | 4-CH₂OBu | 2-Me | H/H |
| CH | O | 4-CF₃ | 4- (CH₂)₅0Me | 2-OMe | Me/Me |
| CH | O | 4-CF₃ | 4-(CH₂)₅0Me | 2-OMe | H/H |
| CH | S | 4-CF₃ | 4-Hex, 5-Me | 2-F | Me/Me |
| CH | S | 4-CF₃ | 4-Hex, 5-Me | 2-F | H/H |
| CH | O | 4-CF₃ | 4-O(CH₂)₅OMe | 2,5-Me | Me/Me |
| CH | O | 4-CF₃ | 4-O(CH₂)₅OMe | 2, 5-Me | H/H |
| N | O | 5-Me | 4-OiPr | 2-Me | Me/Me |
| N | O | 5-Me | 4-OiPr | 2-Me | H/H |

(4) Compounds represented by the following formula: 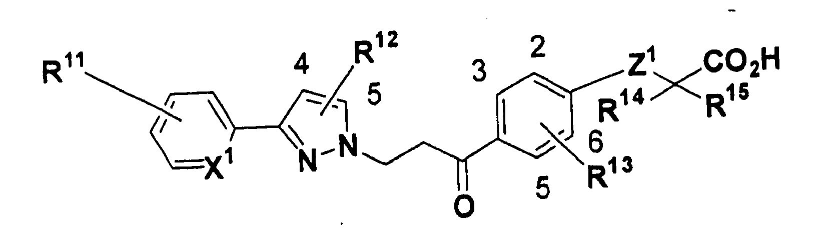 in which each of R¹¹, R¹², R¹³, R¹⁴, R¹⁵, X¹ and Z¹ is that set forth in Tables 7 and 8.

**Table 7**

| X¹ | Z¹ | R¹¹ | R¹² | R¹³ | R¹⁴/R¹⁵ |
|---|---|---|---|---|---|
| CH | O | 4-CF₃ | 5-iPr | 2-Me | Me/Me |
| CH | O | 4-CF₃ | 5-iPr | 2-Me | H/H |
| N | O | 5-CF₃ | 5-iPr | 2-Me | Me/Me |
| N | O | 5-CF₃ | 5-iPr | 2-Me | H/H |
| CH | S | 4-CF₃ | 5-Hex | 3-Me | Me/Me |
| CH | S | 4-CF₃ | 5-Hex | 3-Me | H/H |
| CH | O | 4-Me | 5-Hex | 2-Me | Me/Me |
| CH | O | 4-Me | 5-Hex | 2-Me | H/H |
| CH | O | 4-CF₃ | 4-Me, 5-secBu | 2,5-Me | Me/Me |
| CH | O | 4-CF₃ | 4-Me, 5-secBu | 2,5-Me | H/H |
| CH | O | 4-CF₃ | 5-cyclopropyl | 2,6-Me | Me/Me |

**Table 8**

| X¹ | Z¹ | R¹¹ | R¹² | R¹³ | R¹⁴/R¹⁵ |
|---|---|---|---|---|---|
| CH | O | 4-CF₃ | 5-cyclopropyl | 2,6-Me | H/H |
| N | O | 5-Me | 5-iPr | 2-allyl | Me/Me |
| N | O | 5-Me | 5-iPr | 2-allyl | H/H |
| CH | S | 3,4-OMe | 4-Me, 5-iPr | 2-CF₃ | Me/Me |
| CH | S | 3,4-OMe | 4-Me, 5-iPr | 2-CF₃ | H/H |
| CH | O | 4-CF₃ | 5-CH₂OBu | 2,5-Me | Me/Me |
| CH | O | 4-CF₃ | 5-CH₂OBu | 2, 5-Me | H/H |
| CH | O | 4-CF₃ | 5-(CH₂)₅OMe | 2-OMe | Me/Me |
| CH | O | 4-CF₃ | 5-(CH₂)₅OMe | 2-OMe | H/H |
| CH | S | 4-CF₃ | 5-Hex | 2-F | Me/Me |
| CH | S | 4-CF₃ | 5-Hex | 2-F | H/H |

(5) Compounds represented by the following formula: 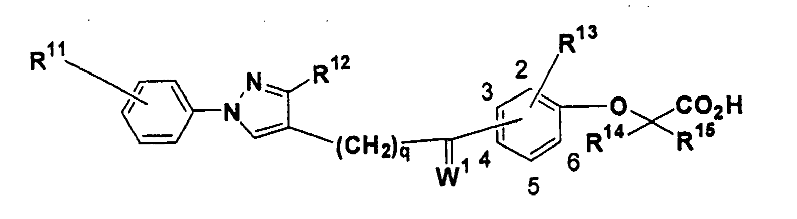 in which each of R¹¹, R¹², R¹³, R¹⁴, R¹⁵, W¹, q and position (position of the benzene ring at which -(CH₂)_{q}-C(=W¹)- is attached) is that set forth in Tables 9 and 10.

**Table 9**

| W¹ | R¹¹ | R¹² | R¹³ | R¹⁴/R¹⁵ | q | position |
|---|---|---|---|---|---|---|
| CH₂ | 4-CF₃ | iPr | 2-Me | Me/Me | 2 | 4 |
| O | 4-CF₃ | iPr | 6-Me | Me/Me | 3 | 3 |
| O | 4-CF₃ | iPr | 6-Me | H/H | 3 | 3 |
| O | 4-Me | Hex | 6-Me | Me/Me | 3 | 3 |
| O | 4-Me | Hex | 6-Me | H/H | 3 | 3 |
| C(Me)₂ | 4-CF₃ | Hex | 2-Me | Me/Me | 2 | 4 |
| C(Me)₂ | 4-CF₃ | Hex | 2-Me | H/H | 2 | 4 |
| C(Me) | 4-Me | Hex | 2-Me | Me/Me | 2 | 4 |
| C (Me) | 4-Me | Hex | 2-Me | H/H | 2 | 4 |
| O | 4-CF₃ | secBu | H | Me/Me | 4 | 4 |
| O | 4-CF₃ | secBu | H | H/H | 4 | 4 |
| O | 4-CF₃ | cyclopropyl | 6-Me | Me/Me | 3 | 3 |
| O | 4-CF₃ | cyclopropyl | 6-Me | H/H | 3 | 3 |
| O | 3,4-Me | iPr | 6-allyl | Me/Me | 3 | 3 |

**Table 10**

| W¹ | R¹¹ | R¹² | R¹³ | R¹⁴/R¹⁵ | q | position |
|---|---|---|---|---|---|---|
| O | 3,4-Me | iPr | 6-allyl | H/H | 3 | 3 |
| O | 4-OMe | iPr | 2-CF₃ | Me/Me | 4 | 4 |
| O | 4-OMe | iPr | 2-CF₃ | H/H | 4 | 4 |
| O | 4-OCF₃ | CH₂OBu | 5-Me | Me/Me | 3 | 3 |
| O | 4-OCF₃ | CH₂OBu | 5-Me | H/H | 3 | 3 |
| O | 4-CF₃ | (CH₂)₅OMe | 2-OMe | Me/Me | 4 | 4 |
| O | 4-CF₃ | (CH₂)₅OMe | 2-OMe | H/H | 4 | 4 |
| O | 4-CF₃ | Hex | 6-F | Me/Me | 3 | 3 |
| O | 4-CF₃ | Hex | 6-F | H/H | 3 | 3 |
| O | 4-CF₃ | O(CH₂)₅Me | 2-Me | Me/Me | 3 | 4 |
| O | 4-CF₃ | O(CH₂)₅Me | 2-Me | H/H | 3 | 4 |
| CH₂ | 4-Me | OiPr | 2,5-Me | Me/Me | 3 | 4 |
| CH₂ | 4-Me | OiPr | 2,5-Me | H/H | 3 | 4 |

(6) Compounds represented by the following formula: 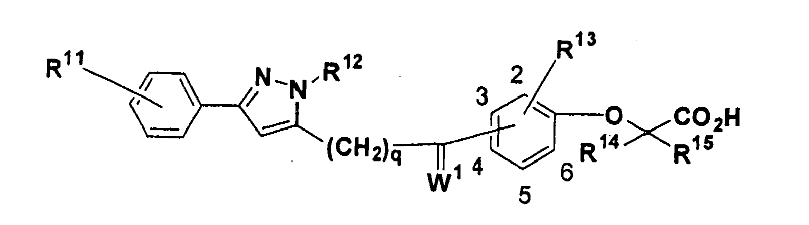 in which each of R¹¹, R¹², R¹³, R¹⁴, R¹⁵, W¹, q and position (position of the benzene ring at which -(CH₂)_{q}-C(=W¹)- is attached) is that set forth in Tables 11 and 12.

**Table 11**

| W¹ | R¹¹ | R¹² | R¹³ | R¹⁴/R¹⁵ | q | position |
|---|---|---|---|---|---|---|
| CH₂ | 4-CF₃ | iPr | 2-Me | Me/Me | 2 | 4 |
| O | 4-CF₃ | iPr | 6-Me | Me/Me | 3 | 3 |
| O | 4-CF₃ | iPr | 6-Me | H/H | 3 | 3 |
| O | 4-Me | Hex | 6-Me | Me/Me | 3 | 3 |
| O | 4-Me | Hex | 6-Me | H/H | 3 | 3 |
| C(Me)₂ | 4-CF₃ | Hex | 2-Me | Me/Me | 2 | 4 |
| C(Me)₂ | 4-CF₃ | Hex | 2-Me | H/H | 2 | 4 |
| C(Me) | 4-Me | Hex | 2-Me | Me/Me | 2 | 4 |
| C(Me) | 4-Me | Hex | 2-Me | H/H | 2 | 4 |
| O | 4-CF₃ | secBu | H | Me/Me | 4 | 4 |
| O | 4-CF₃ | secBu | H | H/H | 4 | 4 |
| O | 4-CF₃ | cyclopropyl | 5,6-Me | Me/Me | 3 | 3 |

**Table 12**

| W¹ | R¹¹ | R¹² | R¹³ | R¹⁴/R¹⁵ | q | position |
|---|---|---|---|---|---|---|
| O | 4-CF₃ | cyclopropyl | 5,6-Me | H/H | 3 | 3 |
| O | 3,4-Me | iPr | 6-allyl | Me/Me | 3 | 3 |
| O | 3,4-Me | iPr | 6-allyl | H/H | 3 | 3 |
| O | 4-OMe | iPr | 2-CF₃ | Me/Me | 4 | 4 |
| O | 4-OMe | iPr | 2-CF₃ | H/H | 4 | 4 |
| O | 4-OCF₃ | (CH₂)₂OBu | 5,6-Me | Me/Me | 3 | 3 |
| O | 4-OCF₃ | (CH₂)₂OBu | 5,6-Me | H/H | 3 | 3 |
| O | 4-CF₃ | (CH₂)₅OMe | 2-Ac | Me/Me | 4 | 4 |
| O | 4-CF₃ | (CH₂)₅OMe | 2-Ac | H/H | 4 | 4 |
| CH₂ | 4-CF₃ | Hex | 6-F | Me/Me | 3 | 3 |
| CH₂ | 4-CF₃ | Hex | 6-F | H/H | 3 | 3 |

(7) Compounds represented by the following formula: 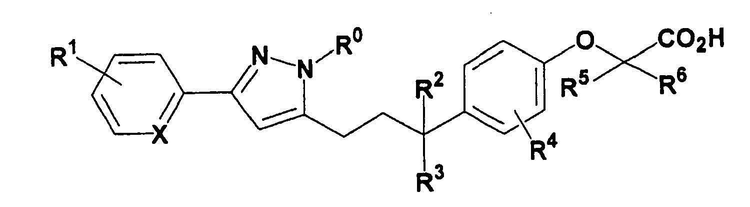 in which R² is H, and each of R¹, R³, R⁴, R⁵, R⁶, R⁰, and X is that set forth in Table 13.

**Table 13**

| X | R¹ | R³ | R⁴ | R⁵/R⁶ | R⁰ |
|---|---|---|---|---|---|
| CH | 4-CF₃ | Me | 2-Me | Me/Me | iPr |
| CH | 4-CF₃ | Me | 2-Me | H/H | iPr |
| N | 5-CF₃ | Et | 2-allyl | Me/Me | iPr |
| N | 5-CF₃ | Et | 2-allyl | H/H | iPr |
| CH | 4-CF₃ | CH(Me)₂ | 2-Me | Me/Me | iPr |
| CH | 4-CF₃ | CH(Me)₂ | 2-Me | H/H | iPr |
| CH | 4-Me | Me | 2,3-Me | Me/Me | Hex |
| CH | 4-Me | Me | 2,3-Me | H/H | Hex |
| N | 5-CF₃ | Et | 2-F | Me/Me | Hex |
| N | 5-CF₃ | Et | 2-F | H/H | Hex |
| CH | 4-Me | CH(Me)₂ | 2,5-Me | Me/Me | Hex |
| CH | 4-Me | CH(Me)₂ | 2,5-Me | H/H | Hex |

(8) Compounds represented by the following formula: 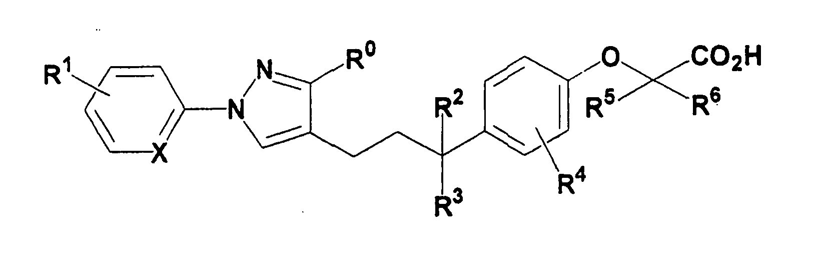 in which R² is H, and each of R¹, R³, R⁴, R⁵, R⁶, R⁰, and X is that set forth in Table 14.

**Table 14**

| X | R¹ | R³ | R⁴ | R⁵/R⁶ | R⁰ |
|---|---|---|---|---|---|
| CH | 4-CF₃ | Me | 2-Me | Me/Me | iPr |
| CH | 4-CF₃ | Me | 2-Me | H/H | iPr |
| N | 5-CF₃ | Et | 2-allyl | Me/Me | iPr |
| N | 5-CF₃ | Et | 2-allyl | H/H | iPr |
| CH | 4-CF₃ | CH(Me)₂ | 2-Me | Me/Me | OEt |
| CH | 4-CF₃ | CH(Me)₂ | 2-Me | H/H | OEt |
| CH | 4-Me | Me | 2-F | Me/Me | Hex |
| CH | 4-Me | Me | 2-F | H/H | Hex |
| N | 5-CF₃ | Et | 2,5-Me | Me/Me | Hex |
| N | 5-CF₃ | Et | 2, 5-Me | H/H | Hex |
| CH | 4-Me | CH(Me)₂ | 2,3-Me | Me/Me | Hex |
| CH | 4-Me | CH(Me)₂ | 2,3-Me | H/H | Hex |

(9) Compounds represented by the following formula: 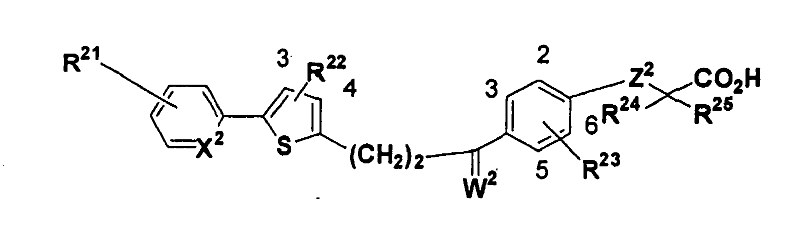 in which each of R²¹, R²² R²³ R²⁴, R²⁵, W², X² and Z² is that set forth in Tables 15 and 16.

**Table 15**

| W² | X² | Z² | R²¹ | R²² | R²³ | R²⁴/R²⁵ |
|---|---|---|---|---|---|---|
| O | CH | O | 4-CF₃ | 4-Me | 2-Me | Me/Me |
| O | CH | O | 4-CF₃ | 4-Me | 2-Me | H/H |
| O | CH | O | 4-CF₃ | 4-iPr | 2-Me | Me/Me |
| O | CH | O | 4-CF₃ | 4-iPr | 2-Me | H/H |
| O | CH | O | 4-CF₃ | 4-Hex | 2-Me | Me/Me |
| O | CH | O | 4-CF₃ | 4-Hex | 2-Me | Me/Me |
| O | CH | O | 4-Me | 4-Hex | 2-Me | Me/Me |
| O | CH | O | 4-Me | 4-Hex | 2-Me | H/H |
| CH₂ | CH | O | 4-CF₃ | 3-Me, 4-secBu | 2,3-Me | Me/Me |
| CH₂ | CH | O | 4-CF₃ | 3-Me, 4-secBu | 2,3-Me | H/H |
| O | CH | O | 4-CF₃ | 4-cyclopropyl | 2,6-Me | Me/Me |
| O | CH | O | 4-CF₃ | 4-cyclopropyl | 2,6-Me | H/H |
| O | N | O | 5-Me | 3-iPr | 2-allyl | Me/Me |
| O | N | O | 5-Me | 3-iPr | 2-allyl | H/H |

**Table 16**

| W² | X² | Z² | R²¹ | R²² | R²³ | R²⁴/R²⁵ |
|---|---|---|---|---|---|---|
| CH(Me) | CH | S | 4-OMe | 3-Me | 2-CF₃ | Me/Me |
| CH(Me) | CH | S | 4-OMe | 3-Me | 2-CF₃ | H/H |
| O | CH | O | 4-CF₃ | 4-CH₂OBu | 2-Me | Me/Me |
| O | CH | O | 4-CF₃ | 4-CH₂OBu | 2-Me | H/H |
| O | CH | O | 4-CF₃ | 4-(CH₂)₅OMe | 2-OMe | Me/Me |
| O | CH | O | 4-CF₃ | 4-(CH₂)₅OMe | 2-OMe | H/H |
| O | CH | S | 4-CF₃ | 3-Me, 4-Hex | 2-F | Me/Me |
| O | CH | S | 4-CF₃ | 3-Me, 4-Hex | 2-F | H/H |
| C(Me)₂ | CH | O | 4-CF₃ | 4-octyl | 2,5-Me | Me/Me |
| C(Me)₂ | CH | O | 4-CF₃ | 4-octyl | 2,5-Me | H/H |
| O | N | O | 5-Me | 4-tBu | 2,5-Me | Me/Me |
| O | N | O | 5-Me | 4-tBu | 2,5-Me | H/H |

(10) Compounds represented by the following formula: 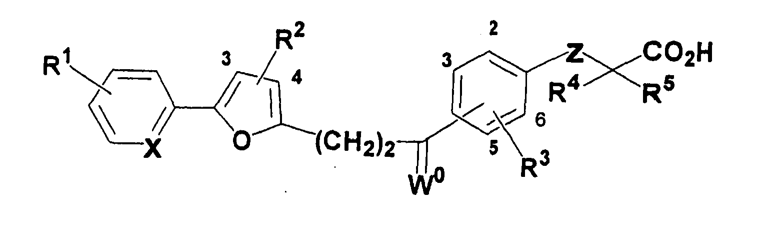 in which each of R¹, R², R³, R⁴, R⁵, W⁰, X and Z is that set forth in Tables 17 and 18.

**Table 17**

| W⁰ | X | Z | R¹ | R² | R³ | R⁴/R⁵ |
|---|---|---|---|---|---|---|
| O | CH | O | 4-CF₃ | 4-iPr | 2-Me | Me/Me |
| O | CH | O | 4-CF₃ | 4-iPr | 2-Me | H/H |
| O | N | O | 5-CF₃ | 4-iPr | 2-Me | Me/Me |
| O | N | O | 5-CF₃ | 4-iPr | 2-Me | H/H |
| O | CH | O | 4-CF₃ | 4-Hex | 2-Me | Me/Me |
| O | CH | O | 4-CF₃ | 4-Hex | 2-Me | H/H |
| CH₂ | CH | O | 3,4-Me | 4-Hex | 2-Me | Me/Me |
| CH₂ | CH | O | 3,4-Me | 4-Hex | 2-Me | H/H |
| O | CH | O | 4-CF₃ | 3-Me, 4-secBu | 2,3-Me | Me/Me |
| O | CH | O | 4-CF₃ | 3-Me, 4-secBu | 2,3-Me | H/H |
| O | CH | O | 4-CF₃ | 4-cyclopropyl | 2,6-Me | Me/Me |
| O | CH | O | 4-CF₃ | 4-cyclopropyl | 2,6-Me | H/H |
| O | N | O | 5-Me | 4-iPr | 2-allyl | Me/Me |

**Table 18**

| W ⁰ | X | Z | R¹ | R² | R³ | R⁴/R⁵ |
|---|---|---|---|---|---|---|
| O | N | O | 5-Me | 4-iPr | 2-allyl | H/H |
| CH(Me) | CH | S | 4-OMe | 3-Me, 4-iPr | 2-CF₃ | Me/Me |
| CH(Me) | CH | S | 4-OMe | 3-Me, 4-iPr | 2-CF₃ | H/H |
| O | CH | O | 4-CF₃ | 4-CH₂OBu | 2-Me | Me/Me |
| O | CH | O | 4-CF₃ | 4-CH₂OBu | 2-Me | H/H |
| O | CH | O | 4-CF₃ | 4-(CH₂)₅OMe | 2-Me | Me/Me |
| O | CH | O | 4-CF₃ | 4-(CH₂)₅OMe | 2-Me | H/H |
| O | CH | S | 4-CF₃ | 3-Me, 4-Hex | 2-F | Me/Me |
| O | CH | S | 4-CF₃ | 3-Me, 4-Hex | 2-F | H/H |
| C(Me)₂ | CH | O | 4-CF₃ | 4-octyl | 2,5-Me | Me/Me |
| C(Me)₂ | CH | O | 4-CF₃ | 4-octyl | 2,5-Me | H/H |
| O | N | O | 5-Me | 4-tBu | 2,5-Me | Me/Me |
| O | N | O | 5-Me | 4-tBu | H | 2,5-Me |

(11) Compounds represented by the following formula: 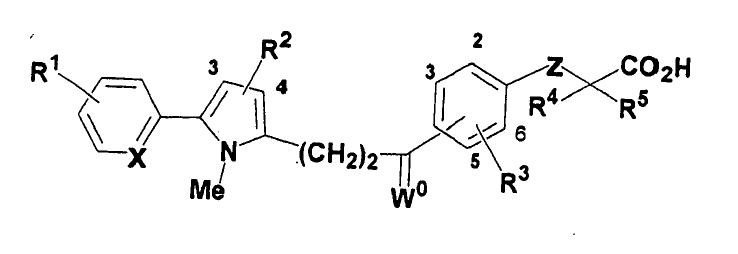 in which each of R¹, R², R³, R⁴, R⁵, W⁰, X and Z is that set forth in Tables 19 and 20.

**Table 19**

| W⁰ | X | Z | R¹ | R² | R³ | R⁴/R⁵ |
|---|---|---|---|---|---|---|
| O | CH | O | 4-CF₃ | 4-iPr | 2-Me | Me/Me |
| O | CH | O | 4-CF₃ | 4-iPr | 2-Me | H/H |
| O | N | O | 5-CF₃ | 4-iPr | 2-Me | Me/Me |
| O | N | O | 5-CF₃ | 4-iPr | 2-Me | H/H |
| O | CH | O | 4-CF₃ | 4-Hex | 2-Me | Me/Me |
| O | CH | O | 4-CF₃ | 4-Hex | 2-Me | H/H |
| CH₂ | CH | O | 4-Me | 4-Hex | 2-Me | Me/Me |
| CH₂ | CH | O | 4-Me | 4-Hex | 2-Me | H/H |
| O | CH | O | 4-CF₃ | 3-Me, 4-secBu | 2,3-Me | Me/Me |
| O | CH | O | 4-CF₃ | 3-Me, 4-secBu | 2,3-Me | H/H |
| O | CH | O | 4-CF₃ | 4-cyclopropyl | 2,6-Me | Me/Me |
| O | CH | O | 4-CF₃ | 4-cyclopropyl | 2,6-Me | H/H |
| O | N | O | 5-Me | 4-iPr | 2-allyl | Me/Me |

**Table 20**

| W⁰ | X | Z | R¹ | R² | R³ | R⁴/R⁵ |
|---|---|---|---|---|---|---|
| O | N | O | 5-Me | 4-iPr | 2-allyl | H/H |
| CH(Me) | CH | S | 4-OMe | 3-Me, 4-iPr | 2-CF₃ | Me/Et |
| CH(Me) | CH | S | 4-OMe | 3-Me, 4-iPr | 2-CF₃ | H/H |
| O | CH | O | 4-CF₃ | 4-CH₂OBu | 2-Me | Me/Me |
| O | CH | O | 4-CF₃ | 4-CH₂OBu | 2-Me | H/H |
| O | CH | O | 4-Et | 4-(CH₂)₅OMe | 2-OMe | Me/Me |
| O | CH | O | 4-Et | 4-(CH₂)₅OMe | 2-OMe | H/H |
| O | CH | S | 4-NO₂ | 3-Me, 4-Hex | 2-F | Me/Me |
| O | CH | S | 4-NO₂ | 3-Me, 4-Hex | 2-F | H/H |
| C(Me)₂ | CH | O | 4-CF₃ | 4-octyl | 2,5-Me | Me/Me |
| C(Me)₂ | CH | O | 4-CF₃ | 4-octyl | 2,5-Me | H/H |
| O | N | O | 5-Me | 4-tBu | 2,5-Me | Me/Me |
| O | N | O | 5-Me | 4-tBu | 2,5-Me | H/H |

(12) Compounds represented by the following formula: 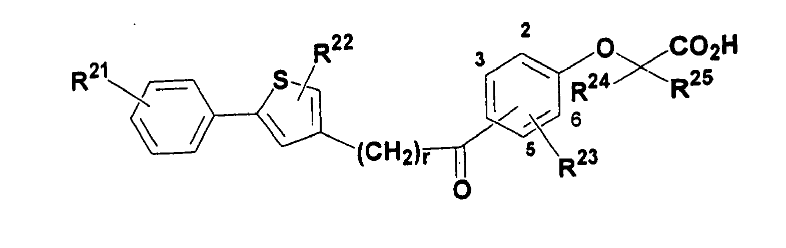 in which each of R²¹, R²², R²³, R²⁴, R²⁵, r and position (position of the benzene ring at which -(CH₂)ᵣ-C(=O)- is attached) is that set forth in Tables 21 and 22.

**Table 21**

| R¹¹ | R²² | R¹³ | R¹⁴/R¹⁵ | r | position |
|---|---|---|---|---|---|
| 4-CF₃ | 5-iPr | 2-Me | Me/Me | 3 | 4 |
| 4-CF₃ | 5-iPr | 2-Me | H/H | 3 | 3 |
| 4-OCF₃ | 5-iPr | 2-Me | Me/Me | 3 | 4 |
| 4-OCF₃ | 5-iPr | 6-Me | H/H | 3 | 3 |
| 4-CF₃ | 5-Hex | 2-Me | Me/Me | 3 | 4 |
| 4-CF₃ | 5-Hex | 6-Me | H/H | 3 | 3 |
| 4-Me | 5-Hex | 2-Me | Me/Me | 3 | 4 |
| 4-Me | 5-Hex | 6-Me | H/H | 3 | 3 |
| 4-CF₃ | 3-Me, 5-secBu | 4,5-Me | Me/Me | 2 | 3 |
| 4-CF₃ | 3-Me, 5-secBu | 4,5-Me | H/H | 2 | 3 |
| 4-CF₃ | 5-cyclopropyl | 4,6-Me | Me/Me | 2 | 3 |
| 4-CF₃ | 5-cyclopropyl | 4,6-Me | H/H | 2 | 3 |
| 3,4-Me | 5-iPr | 6-allyl | Me/Me | 2 | 3 |

**Table 22**

| R¹¹ | R²² | | R¹³ | R¹⁴/R¹⁵ | r | position |
|---|---|---|---|---|---|---|
| 3,4-Me | 5-iPr | | 6-allyl | H/H | 2 | 3 |
| 4-OMe | 3-Me, | 5-iPr | 6-CF₃ | Me/Me | 2 | 3 |
| 4-OMe | 3-Me, | 5-iPr | 2-CF₃ | H/H | 3 | 4 |
| 4-CF₃ | 5-CH₂OBu | | 2-Me | Me/Me | 3 | 4 |
| 4-CF₃ | 5-CH₂OBu | | 6-Me | H/H | 3 | 3 |
| 4-CF₃ | 5-(CH₂)₅OMe | | 2-OMe | Me/Me | 3 | 4 |
| 4-CF₃ | 5-(CH₂)₅OMe | | 6-OMe | H/H | 3 | 3 |
| 4-CF₃ | 3-Me, 5-Hex | | 2-F | Me/Me | 4 | 4 |
| 4-CF₃ | 3-Me, 5-Hex | | 6-F | H/H | 4 | 3 |
| 4-CF₃ | 5-octyl | | 2,5-Me | Me/Me | 3 | 4 |
| 4-CF₃ | 5-octyl | | 2,5-Me | H/H | 3 | 3 |
| 4-OMe | 5-tBu | | 2,5-Me | Me/Me | 3 | 4 |
| 4-OMe | 5-tBu | | 2,5-Me | H/H | 3 | 3 |

(13) Compounds represented by the following formula: 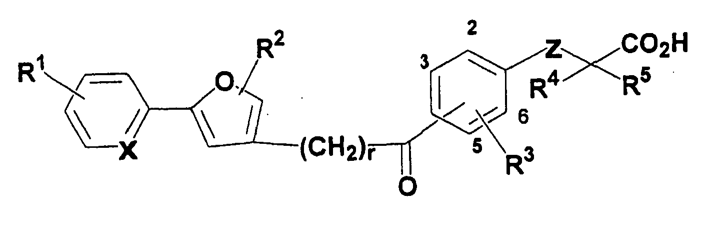 in which each of R¹, R², R³, R⁴, R⁵, r and position (position of the benzene ring at which -(CH₂)ᵣ-C(=O)- is attached) is that set forth in Tables 23 and 24.

**Table 23**

| R¹ | R² | R³ | R⁴/R⁵ | r | position |
|---|---|---|---|---|---|
| 4-CF₃ | 5-iPr | 2-Me | Me/Me | 3 | 4 |
| 4-CF₃ | 5-iPr | 2-Me | H/H | 3 | 3 |
| 4-OCF₃ | 5-iPr | 2-Me | Me/Me | 3 | 4 |
| 4-OCF₃ | 5-iPr | 6-Me | H/H | 3 | 3 |
| 4-CF₃ | 5-Hex | 2-Me | Me/Me | 3 | 4 |
| 4-CF₃ | 5-Hex | 6-Me | H/H | 3 | 3 |
| 4-Me | 5-Hex | 2-Me | Me/Me | 3 | 4 |
| 4-Me | 5-Hex | 6-Me | H/H | 3 | 3 |
| 4-CF₃ | 3-Me, 5-secBu | 5,6-Me | Me/Me | 2 | 3 |
| 4-CF₃ | 3-Me, 5-secBu | 5,6-Me | H/H | 2 | 3 |
| 4-CF₃ | 5-cyclopropyl | 5,6-Me | Me/Me | 2 | 3 |
| 4-CF₃ | 5-cyclopropyl | 5,6-Me | H/H | 2 | 3 |
| 3,4-Me | 5-iPr | 6-allyl | Me/Me | 2 | 3 |

**Table 24**

| R¹¹ | R²² | R¹³ | R¹⁴/R¹⁵ | r | position |
|---|---|---|---|---|---|
| 3,4-Me | 5-iPr | 6-allyl | H/H | 2 | 3 |
| 4-OMe | 3-Me, 5-iPr | 6-CF₃ | Me/Me | 2 | 3 |
| 4-OMe | 3-Me, 5-iPr | 2-CF₃ | H/H | 3 | 4 |
| 4-CF₃ | 5-CH₂OBu | 2-Me | Me/Me | 3 | 4 |
| 4-CF₃ | 5-CH₂OBu | 6-Me | H/H | 3 | 3 |
| 4-CF₃ | 5-(CH₂)₅OMe | 2-OMe | Me/Me | 3 | 4 |
| 4-CF3 | 5-(CH₂)₅OMe | 6-OMe | H/H | 3 | 3 |
| 4-CF₃ | 3-Me, 5-Hex | 2-F | Me/Me | 4 | 4 |
| 4-CF₃ | 3-Me, 5-Hex | 6-F | H/H | 4 | 3 |
| 4-CF₃ | 5-octyl | 2,5-Me | Me/Me | 3 | 4 |
| 4-CF₃ | 5-octyl | 5,6-Me | H/H | 3 | 3 |
| 4-OMe | 5-tBu | 2,5-Me | Me/Me | 3 | 4 |
| 4-OMe | 5-tBu | 4,5-Me | H/H | 3 | 3 |

(14) Compounds represented by the following formula: 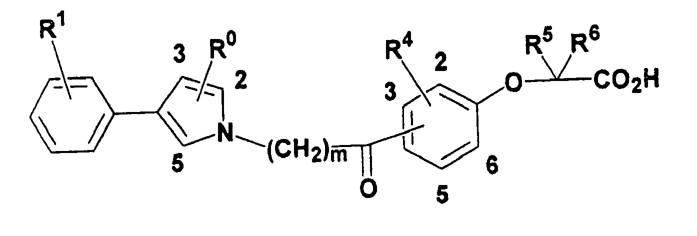 in which each of R¹, R⁰, R⁴, R⁵, R⁶, m and position (position of the benzene ring at which -(CH₂)ₘ-C(=O)- is attached) is that set forth in Tables 25 and 26.

**Table 25**

| R¹ | R⁰ | R⁴ | R⁵/R⁶ | m | position |
|---|---|---|---|---|---|
| 4-CF₃ | 2-iPr | 2-Me | Me/Me | 3 | 4 |
| 4-CF₃ | 2-iPr | 6-Me | H/H | 3 | 3 |
| 4-OCF₃ | 2-iPr | 2-Me | Me/Me | 3 | 4 |
| 4-OCF₃ | 2-iPr | 6-Me | H/H | 3 | 3 |
| 4-CF₃ | 2-Hex | 2-Me | Me/Me | 3 | 4 |
| 4-CF₃ | 2-Hex | 2-Me | H/H | 3 | 3 |
| 4-Me | 2-Hex | 2-Me | Me/Me | 3 | 4 |
| 4-Me | 2-Hex | 6-Me | H/H | 3 | 3 |
| 4-CF₃ | 2-secBu, 3-Me | 6-Me | Me/Me | 2 | 3 |
| 4-CF₃ | 2-secBu, 3-Me | 6-Me | H/H | 2 | 3 |
| 4-CF₃ | 2-cyclopropyl | 6-Me | Me/Me | 2 | 3 |
| 4-CF₃ | 2-cyclopropyl | 6-Me | H/H | 2 | 3 |
| 3,4-Me | 5-iPr | 6-allyl | Me/Me | 2 | 3 |

**Table 26**

| R¹ | R⁰ | R⁴ | R⁵/R⁶ | m | position |
|---|---|---|---|---|---|
| 3,4-Me | 2-iPr | 6-allyl | H/H | 2 | 3 |
| 4-OMe | 2-iPr, 3-Me | 2-CF₃ | Me/Me | 2 | 3 |
| 4-OMe | 2-iPr, 3-Me | 2-CF₃ | H/H | 3 | 4 |
| 4-CF₃ | 2-CH₂OBu, 5-Me | 2-Me | Me/Me | 3 | 4 |
| 4-CF₃ | 2-CH₂OBu, 5-Me | 6-Me | H/H | 3 | 3 |
| 4-CF₃ | 2-(CH₂)₅OMe | 2-OMe | Me/Me | 3 | 4 |
| 4-CF₃ | 2-(CH₂)₅OMe | 6-OMe | H/H | 3 | 3 |
| 4-CF₃ | 2-Hex, 3-Me | 2-F | Me/Me | 4 | 4 |
| 4-CF₃ | 2-Hex, 3-Me | 6-F | H/H | 4 | 3 |
| 4-CF₃ | 2-octyl | 2,5-Me | Me/Me | 3 | 4 |
| 4-CF₃ | 2-octyl | 2,5-Me | H/H | 3 | 3 |
| 4-OMe | 2-tBu | 2,5-Me | Me/Me | 3 | 4 |
| 4-OMe | 2-tBu | 2,5-Me | H/H | 3 | 3 |

The pharmacological effects of the invention are described below.

For determining PPARδ activating effect of the compounds according to the invention, PPARδ activating effects of test compounds (compounds of Examples) were measured by the following method:
A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL)(Kliewer, S.A., et. al.,(1992) Nature, 358:771-774) are transfected into CV-1 cells (ATCC). After gene transfer utilizing a lipofection reagent DMRIE-C or Lipofectamin 2000 (Invitrogen), it is incubated for 42 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions: a relative activity of PPAR α is calculated in consideration of a luciferase activity (assigned to 100%) of cells treated with the compound described in Example 2 of the aforementioned Patent Publication 4 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516. See the below-described Examples 22 and 23.

As is apparent from Tables 27 and 28, the compounds of the invention show excellent PPARδ activating effect.

Since the compound of the invention having the formula (I), (II) or (III) shows excellent PPARδ activating effect, it is expected to serve as remedy for prevention and treatment of the following diseases: hyperglycemia, obesity, syndrome X, hyperchloresterolemia, hyperlipopreoteinemia, other dysbolismic diseases, hiperlipemia, arterial sclerosis, diseases of cardiovascular systems, hyperphagia, ischemic diseases, malignant tumors such as lung cancer, mammary cancer, colonic cancer, cancer of great intestine abd ovary cancer, Alzheimer's disease, and inflammatory disease.

The compound of the invention can be administered to human beings by ordinary administration methods such as oral administration or parenteral administration.

The compound can be granulated in ordinary manners for the preparation of pharmaceuticals. For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents. As the vehicles, lactose, D-mannitol, crystalline cellulose and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinyl-pirrolidone (PVP) as the binders.

The compound of the invention can be administered to an adult generally in an amount of 0.1 mg to 100 mg a day by parenteral administration and 1 mg to 2,000 mg a day by oral administration. The dosage can be adjusted in consideration of age and conditions of the patient.

The invention is further described by the following non-limiting examples.

### [Example 1] 2-[4-[3-[3-Isopropyl-1-(4-trifluoromethylphenyl)-1H-pyrazol-4-yl]propionyl]-2-methylphenoxy]-2-methylpropionic acid

### (1) 1-(4-Hydroxy-3-methylphenyl)-3-[3-isopropyl-1-(4-trifluoromethylphenyl)-1H-pyrazol-4-yl]propan-1-one

To a solution of [3-isopropyl-1-(4-trifluoromethylphenyl)-1H-pyrazol-4-yl]methanol (224 mg, 0.788 mmol) in benzene (2.5 mL) was dropwise added a solution of thionyl chloride (0.07 mL, 0.946 mmol) in benzene (1.5 mL) under cooling with ice. The resulting mixture was stirred for 3 hours at room temperature and placed under reduced pressure to distill the solvent off, to yield 4-chloromethyl-3-isopropyl-1-(4-trifluoromethylphenyl)-1H-pyrazole (240 mg) as a residue.

Subsequently, 55% sodium hydride (38 mg, 0.869 mmol) was suspended in anhydrous tetrahydrofuran (10 mL) under nitrogen atmosphere. To the suspension was dropwise added a solution of ethyl 3-(4-benzyloxy-3-methylphenyl)-3-oxopropionate (247 mg, 0.790 mmol) in anhydrous tetrahydrofuran (3 mL) under cooling with ice for 10 minutes. After 30 minutes, to the resulting solution was added a solution of the above-mentioned 4-chloromethyl-3-isopropyl-1-(4-trifluoromethylphenyl)-1H-pyrazole (240 mg) in anhydrous tetrahydrofuran (3 mL) for 15 minutes. The resulting mixture was then heated under reflux for 23 hours. The reaction mixture was cooled to room temperature and concentrated under reduced pressure, to yield a residue. To the residue were added acetic acid (5.2 mL) and concentrated hydrochloric acid (1 mL). The resulting mixture was stirred at 110°C for 20 hours and then cooled to room temperature. The cooled mixture was mixed with aqueous saturated sodium hydrogencarbonate (30 mL) and then subjected to extraction with ethyl acetate (50 mL). The organic portion was collected, washed with aqueous saturated sodium hydrogencarbonate (30 mL x 3), saturated brine (30 mL) and water (30 mL), and dried over anhydrous sodium sulfate. The dried extract was placed under reduced pressure to distill the solvent off. The resulting residue was subjected to silica gel column chromatography. The fraction obtained by elution with hexane/ethyl acetate (6/1, v/v) gave the titled compound as a white crystalline product (213 mg, yield 65%).
¹H NMR (CDCl₃, 400 MHz) δ: 1.35 (6H, d, J=7Hz), 2.29 (3H, s), 2.94 (2H, t, J=7Hz), 3.0-3.1 (1H, m), 3.22 (2H, t, J=7Hz), 5.21 (1H, s), 6.81 (1H, d, J=8Hz), 7.63 (2H, d, J=8Hz), 7.74 (2H, d, J=8Hz), 7.7-7.8 (3H, m)

### (2) Ethyl 2-[4-[3-[3-isopropyl-1-(4-trifluoromethylphenyl)-1H-pyrazol-4-yl]propionyl]-2-methylphenoxy]-2-methylpropionate

To a suspension of the above-mentioned 1-(4-hydroxy-3-methylphenyl)-3-[3-isopropyl-1-(4-trifluoromethylphenyl)-1H-pyrazol-4-yl]propan-1-one (100 mg, 0.240 mmol) and potassium carbonate (166 mg, 1.20 mmol) in 2-butanone (3 mL) was added ethyl 2-bromoisobutyrate (0.18 mL, 1.20 mmol). The resulting mixture was stirred under heating for 13 hours, and cooled to room temperature. The cooled mixture was mixed with aqueous saturated ammonium chloride (10 mL) and subjected to extraction with ethyl acetate (30 mL x 2). The organic portion was collected, washed with saturated brine (30 mL), and dried over anhydrous sodium sulfate. The dried extract was placed under reduced pressure to distill the solvent off. The residue was subjected to silica gel column chromatography. The fraction obtained by elution with hexane/ethyl acetate (15/1, v/v) gave the titled compound as a colorless oily product (119 mg, yield 93%).
¹H NMR (CDCl₃, 400 MHz) δ: 1.22 (3H, t, J=7Hz), 1.35 (6H, d, J=7Hz), 1.65 (6H, s), 2.27 (3H, s), 2.93 (2H, t, J=7Hz), 3.0-3.1 (1H, m), 3.21 (2H, t, J=7Hz), 4.22 (2H, q, J=7Hz), 6.62 (1H, d, J=9Hz), 7.64 (2H, d, J=9Hz), 7.7-7.8 (2H, m), 7.74 (2H, d, J=9Hz), 7.80 (1H, d, J=1Hz)

### (3) 2-[4-[3-[3-Isopropyl-1-(4-trifluoromethylphenyl)-1H-pyrazol-4-yl]propionyl]-2-methylphenoxy]-2-methylpropionic acid

The above-mentioned ethyl 2-[4-[3-[3-isopropyl-1-(4-trifluoromethylphenyl)-1H-pyrazol-4-yl]propionyl]-2-methylphenoxy]-2-methylpropionate (119 mg, 0.224 mmol) was dissolved in a mixture of ethanol (2 mL) and water (1 mL). To the solution was added lithium hydroxide monohydrate (28 mg, 0.673 mmol). The resulting mixture was heated under reflux for one hour and cooled to room temperature. The cooled mixture was acidified by addition of ice-water and 1N hydrochloric acid, and subjected to extraction with ethyl acetate (10 mL). The organic portion was collected, washed with saturated brine, and dried over anhydrous sodium sulfate. The dried extract was placed under reduced pressure to distill the solvent off. The residue was subjected to silica gel column chromatography. The fraction obtained by elution with chloroform/methanol (20/1, v/v) gave the titled compound as a pale yellow amorphous product (86 mg, yield 76%).
FAB-MS (m/e): 503 (M+1)
¹H NMR (CDCl₃, 400 MHz) δ: 1.33 (6H, d, J=7Hz), 1.67 (6H, s), 2.26 (3H, s), 2.92 (2H, t, J=7Hz), 3.0-3.1 (1H, m), 3.21 (2H, t, J=7Hz), 6.73 (1H, d, J=8Hz), 7.62 (2H, d, J=9Hz), 7.7-7.8 (2H, m), 7.72 (2H, d, J=9Hz), 7.80 (1H, d, J=1Hz)

### [Example 2] 4-[3-[3-Isopropyl-1-(4-trifluoromethylphenyl)-1H-pyrazol-4-yl]propionyl]-2-methylphenoxyacetic acid

### (1) Ethyl 4-[3-[3-isopropyl-1-(4-trifluoromethylphenyl)-1H-pyrazol-4-yl]propionyl]-2-methylphenoxyacetate

To a solution of 1-(4-hydroxy-3-methylphenyl)-3-[isopropyl-1-(4-trifluoromethylphenyl)-1H-pyrazol-4-yl]-propane-1-one (110 mg, 0.264 mmol) and potassium carbonate (73 mg, 0.528 mmol) in 2-butanone (1.8 mL) was slowly added ethyl bromoacetate (0.06 mL, 0.528 mmol) under cooling with ice. The mixture was stirred for 2 hours at room temperature, mixed with aqueous saturated ammonium chloride (10 mL), and then subjected to extraction with ethyl acetate (30 mL x 2). The organic portion was collected, washed with saturated brine (30 mL), and dried over anhydrous sodium sulfate. The dried extract was placed under reduced pressure to distill the solvent off. The residue was subjected to silica gel column chromatography. The fraction obtained by elution with hexane/ethyl acetate (12/1, v/v) gave the titled compound as a colorless oily product (122 mg, yield 92%).
¹H NMR (CDCl₃, 400 MHz) δ: 1.30 (3H, t, J=7Hz), 1.35 (6H, d, J=7Hz), 2.33 (3H, s), 2.94 (2H, t, J=7Hz), 3.0-3.1 (1H, m), 3.23 (2H, t, J=7Hz), 4.27 (2H, q, J=7Hz), 4.71 (2H, s), 6.72 (1H, d, J=8Hz), 7.64 (2H, d, J=9Hz), 7.71 (1H, s), 7.74 (2H, d, J=9Hz), 7.8-7.9 (1H, m), 7.81 (1H, s)

### (2) 4-[3-[3-Isopropyl-1-(4-trifluoromethylphenyl)-1H-pyrazol-4-yl]propionyl]-2-methylphenoxyacetic acid

The titled compound was prepared by procedures similar to the procedures of Example 1-(3).

White crystalline product
Yield 81%
FAB-MS (m/e): 475 (M+1)
¹H NMR (CDCl₃, 400 MHz) δ: 1.35 (6H, d, J=7Hz), 2.33 (3H, s), 2.93 (2H, t, J=7Hz), 3.0-3.1 (1H, m), 3.23 (2H, t, J=7Hz), 4.76 (2H, s), 6.74 (1H, d, J=9Hz), 7.64 (2H, d, J=9Hz), 7.71 (1H, s), 7.74 (2H, d, J=9Hz), 7.8-7.9 (1H, m), 7.82 (1H, s)

### [Example 3] 2-[4-[3-[3-Isopropyl-1-(5-methylpyridin-2-yl)-1H-pyrazol-4-yl]propionyl]-2-methylphenoxy]-2-methylpropionic acid

### (1) 1-(4-Hydroxy-3-methylphenyl)-3-[3-isopropyl-1-(5-methylpyridin-2-yl)-1H-pyrazol-4-yl]propan-1-one

The procedures of Example 1-(1) were repeated except for employing [3-isopropyl-1-(5-methylpyridin-2-yl)-1H-pyrazol-4-yl]methanol, to yield the titled compound.
¹H NMR (CDCl₃, 400 MHz) δ: 1.35 (6H, d, J=7Hz), 2.30 (3H, s), 2.33 (3H, s), 2,91 (2H, t, J=7Hz), 3.0-3.2 (1H, m), 3.25 (2H, t, J=7Hz), 6.80 (1H, d, J=8Hz), 7.56 (1H, dd, J=2, 8Hz), 7.76 (1H, dd, J=2, 8Hz), 7.81 (1H, d, J=2Hz), 7.82 (1H, d, J=8Hz), 8.16 (1H, d, J=2Hz), 8.24 (1H, s)

### (2) Ethyl 2-[4-[3-[3-isopropyl-1-(5-methylpyridin-2-yl)-1H-pyrazol-4-yl]propionyl]-2-methylphenoxy]-2-methylpropionate

The titled compound was prepared by procedures similar to the procedures of Example 1-(2).

¹H NMR (CDCl₃, 400 MHz) δ: 1.22 (3H, t, J=7Hz), 1.34 (6H, d, J=7Hz), 1.66 (6H, s), 2.27 (3H, s), 2.33 (3H, s), 2.91 (2H, t, J=7Hz), 3.0-3.2 (1H, m), 3.25 (2H, t, J=7Hz), 4.23 (2H, q, J=7Hz), 6.62 (1H, d, J=8Hz), 7.56 (1H, dd, J=2, 8Hz), 7.69 (1H, dd, J=2, 8Hz), 7.78 (1H, d, J=2Hz), 7.82 (1H, d, J=8Hz), 8.16 (1H, d, J=2Hz), 8.24 (1H, s)

### (3) 2-[4-[3-[3-Isopropyl-1-(5-methylpyridin-2-yl)-1H-pyrazol-4-yl]propionyl]-2-methylphenoxy]-2-methylpropionic acid

The titled compound was prepared by procedures similar to the procedures of Example 1-(3).

Pale yellow oily product
FAB-MS (m/e): 450 (M+1)
¹H NMR (CDCl₃, 400 MHz) δ: 1.32 (6H, d, J=7Hz), 1.68 (6H, s), 2.25 (3H, s), 2.32 (3H, s), 2.86 (2H, t, J=7Hz), 3.0-3.1 (1H, m), 3.18 (2H, t, J=7Hz), 6.74 (1H, d, J=8Hz), 7. 57 (1H, dd, J=2, 9Hz), 7.71 (1H, dd, J=2, 8Hz), 7.76 (1H, d, J=2Hz), 7.81 (1H, d, J=9Hz), 8.18 (1H, d, J=2Hz), 8.19 (1H, s)

### [Example 4] 4-[3-[3-Isopropyl-1-(5-methylpyridin-2-yl)-1H-pyrazol-4-yl]propionyl]-2-methylphenoxyacetic acid

### (1) Ethyl 4-[3-[3-isopropyl-1-(5-methylpyridin-2-yl)-1H-pyrazol-4-yl]propionyl]-2-methylphenoxyacetate

The titled compound was prepared by procedures similar to the procedures of Example 2-(1).

¹H NMR (CDCl₃, 400 MHz) δ: 1.30 (3H, t, J=7Hz), 1.35 (6H, d, J=7Hz), 2.33 (3H, s), 2.91 (2H, t, J=8Hz), 3.0-3.2 (1H, m), 3.26 (2H, t, J=8Hz), 4.27 (2H, q, J=7Hz), 4.71 (2H, s), 6.72 (1H, d, J=8Hz), 7.56 (1H, dd, J=2, 8Hz), 7.8-7.9 (3H, m), 8.16 (1H, d, J=2Hz), 8.24 (1H, s)

### (2) 4-[3-[3-Isopropyl-1-(5-methylpyridin-2-yl)-1H-pyrazol-4-yl]propionyl]-2-methylphenoxyacetic acid

The titled compound was prepared by procedures similar to the procedures of Example 2-(2).
White crystalline product
FAB-MS (m/e): 422 (M+1)
¹H NMR (CDCl₃, 400 MHz) δ: 1.35 (6H, d, J=7Hz), 2.32 (3H, s), 2.34 (3H, s), 2.91 (2H, t, J=7Hz), 3.0-3.2 (1H, m), 3.28 (2H, t, J=7Hz), 4.71 (2H, s), 6.73 (1H, d, J=9Hz), 7.59 (1H, dd, J=2, 9Hz), 7.8-7.9 (3H, m), 8.19 (1H, d, J=2Hz), 8.25 (1H, s)

### [Example 5] 2-[4-[3-[1-Isopropyl-3-(4-trifluoromethylphenyl)-1H-pyrazol-5-yl]propionyl]-2-methylphenoxy]-2-methylpropionic acid

### (1) Ethyl 1-isopropyl-3-(4-trifluoromethylphenyl)-1H-pyrazol-5-carboxylate

To a solution of ethyl 3-(4-trifluoromethylphenyl)-1H-pyrazol-5-carboxylate (2.85 g, 10.0 mmol) in acetone (50 mL) was added potassium carbonate (1.66 g, 12.0 mmol). The mixture was stirred for 20 minutes at room temperature. Subsequently, 2-iodopropane (1.20 mL, 12.0 mmol) was added. The resulting mixture was heated under reflux for 5 hours and cooled to room temperature. The cooled mixture was mixed with ice-water (100 mL) and subjected to extraction with methylene chloride (100 mL, 50 mL). The extract was dried over anhydrous sodium sulfate and placed under pressure to distill the solvent off. The residue was purified by silica gel column chromatography (ethyl acetate/hexane=1/10), to give the titled compound as a pale brown crystalline product (2.17 g, yield 66%).

¹H NMR (CDCl₃, 400 MHz) δ: 1.41 (3H, t, J=7Hz), 1.55 (6H, d, J=6Hz), 4.37 (2H, q, J=7Hz), 5.5-5.6 (1H, m), 7.16 (1H, s), 7.64 (2H, d, J=8Hz), 7.94 (2H, d, J=8Hz)

### (2) [1-Isopropyl-3-(4-trifluoromethylphenyl)-1H-pyrazol-5-yl]methanol

Lithium aluminum hydride (247 mg, 6.51 mmol) was suspended in dry tetrahydrofuran (50 mL) under a nitrogen atmosphere. To the resulting dispersion was dropwise added a solution of the above-mentioned ethyl 1-isopropyl-3-(4-trifluoromethylphenyl)-1H-pyrazol-5-carboxylate (2.12 g, 6.50 mmol) in dry tetrahydrofuran (50 mL) for 15 minutes under cooling with ice. The mixture was then stirred for one hour at room temperature. Subsequently, aqueous saturated sodium sulfate and ethyl acetate (100 mL) were added under cooling with ice. The organic portion was collected, washed with saturated brine (50 mL), and dried over anhydrous sodium sulfate. The dried extract was placed under reduced pressure, to give the titled compound as a white crystalline product (1.85 g, yield 1.00%).
¹H NMR (CDCl₃, 400 MHz) δ: 1.55 (6H, d, J=6Hz), 1.71 (1H, t, J=5Hz), 4.6-4.7 (1H, m), 4.72 (2H, d, J=5Hz), 6.51 (1H, s), 7.62 (2H, d, J=8Hz), 7.89 (2H, d, J=8Hz)

### (3) 1-(4-Hydroxy-4-methylphenyl)-3-[1-isopropyl-3-(4-trifluoromethylphenyl)-1H-pyrazol-5-yl]propan-1-one

To a solution of the above-mentioned [1-isopropyl-3-(4-trifluoromethylphenyl)-1H-pyrazol-5-yl]methanol (1.77 g, 6.23 mmol) in dry methylene chloride (70 mL) was added phosphorus tribromide (0.22 mL, 2.08 mmol) under cooling with ice. The resulting mixture was stirred for one hour at room temperature. The reaction mixture was poured into aqueous saturated sodium carbonate (100 mL) and subjected to extraction with methylene chloride (150 mL x 2). The organic portion was collected, washed with water (100 mL x 3) and saturated brine (100 mL), and dried over anhydrous sodium sulfate. The dried extract was placed under reduced pressure to distill the solvent off, to give 5-bromomethyl-1-isopropyl-3-(4-trifluoromethylphenyl)-1H-pyrazole as a pale yellow oily product (1.84 g, yield 85%).

Separately, 60% sodium hydride (116 mg, 2.90 mmol) was suspended in dry tetrahydrofuran (10 mL) under a nitrogen atmosphere. Under cooling ice, a solution of ethyl 3-(4-benzyloxy-3-methylphenyl)-3-oxopropionate (900 mg, 2.28 mmol) in dry tetrahydroxyfuran (5 mL) was dropwise added. The mixture was stirred for 20 minutes at room temperature. A solution of the above-mentioned 5-bromomethyl-1-isopropyl-3-(4-trifluoromethylphenyl)-1H-pyrazole (1.00 g, 2.88 mmol) in dry tetrahydrofuran (10 mL) was added. The resulting mixture was heated under reflux for 16 hours and then cooled to room temperature. The reaction mixture was concentrated under reduced pressure. The residue was mixed with acetic acid (8 mL) and concentrated hydrochloric acid (2 mL). The mixture was stirred at 100°C for 7 hours, and then cooled to room temperature. The cooled mixture was adjusted to pH 7 by adding ice-water (50 mL) and aqueous saturated sodium hydrogencarbonate. The resulting mixture was subjected to extraction with ethyl acetate (100 mL x 2). The organic portion was washed with water (50 mL) and dried over anhydrous sodium sulfate. The dried extract was placed under reduced pressure to distill the solvent off. The residue was purified by silica gel column chromatography (methanol/chloroform = 1/100 to 1/20), to give the titled compound as a white crystalline product (951 mg, yield 79%).
¹H NMR (DMSO-D₆, 400 MHz) δ: 1.45 (6H, d, J=6Hz), 2.17 (3H, s), 2.98 (2H, t, J=7Hz), 3.37 (2H, t, J=7Hz), 4.6-4.7 (1H, m), 6.65 (1H, s), 6.86 (1H, d, J=9Hz), 7.71 (2H, d, J=8Hz), 7.75 (1H, dd, J=2, 9Hz), 7.80 (1H, d, J=2Hz), 7.94 (2H, d, J=8Hz), 10.24 (1H, s)

### (4) Ethyl 2-[4-[3-[1-isopropyl-3-(4-trifluoromethylphenyl)-1H-pyrazol-5-yl]propionyl]-2-methylphenoxy]-2-methylpropionate

To a suspension of the above-mentioned 1-(4-hydroxy-4-methylphenyl)-3-[1-isopropyl-3-(4-trifluoromethylphenyl)-1H-pyrazol-5-yl]propane-1-one (150 mg, 0.360 mmol) and potassium carbonate (359 mg, 2.60 mmol) in 2-butanone (10 mL) was added ethyl 2-bromoisobutyrate (0.38 mL, 2.59 mmol). The resulting mixture was heated under reflux for 32 hours, and then cooled to room temperature. The insolubles were filtered off. The filtrate was concentrated under reduced pressure. The residue was purified by silica gel column chromatography (ethyl acetate/hexane = 1/10), to give the titled compound as a white crystalline product (179 mg, yield 94%).
¹H NMR (CDCl₃, 400 MHz) δ: 1.22 (3H, t, J=7Hz), 1.54 (6H, d, J=6Hz), 1.66 (6H, s), 2.28 (3H, s), 3.08 (2H, t, J=7Hz), 3.32 (2H, t, J=7Hz), 4.23 (2H, q, J=7Hz), 4.5-4.6 (1H, m), 6.36 (1H, s), 6.63 (1H, d, J=8Hz), 7.60 (2H, d, J=8Hz), 7.74 (1H, dd, J=2, 8Hz), 7.82 (1H, d, J=2Hz), 7.88 (2H, d, J=8Hz)

### (5) 2-[4-[3-[1-Isopropyl-3-(4-trifluoromethylphenyl)-1H-pyrazol-5-yl]propionyl]-2-methylphenoxy]-2-methylpropionic acid

To a solution of the above-mentioned ethyl 2-[4-[3-[1-isopropyl-3-(4-trifluoromethylphenyl)-1H-pyrazol-5-yl]propionyl]-2-methylphenoxy]-2-methylpropionate (169 mg, 0.32 mmol) in a mixture of ethanol (10 mL) and tetrahydrofuran (5 mL) was added 2M aqueous sodium hydroxide (0.96 mL, 1.92 mmol) under cooling with ice. The resulting mixture was stirred for 24 hours at room temperature. The reaction mixture was concentrated under reduced pressure. The residue was adjusted to pH 3 by adding ice-water and 1M aqueous hydrochloric acid. The mixture was then subjected to extraction with ethyl acetate (50 mL x 2). The organic portion was washed with water (30 mL) and saturated brine (30 mL) and dried over anhydrous sodium sulfate. The dried extract was placed under reduced pressure to distill the solvent off, to give the titled compound as a white amorphous product (121 mg, yield 76%).
FAB-MS (m/e): 503 (M+1)
¹H NMR (CDCl₃, 400 MHz) δ: 1.54 (6H, d, J=7Hz), 1.70 (6H, s), 2.29 (3H, s), 3.08 (2H, t, J=7Hz), 3.33 (2H, t, J=7Hz), 4.5-4.6 (1H, m), 6.35 (1H, s), 6.77 (1H, d, J=8Hz), 7.60 (2H, d, J=8Hz), 7.76 (1H, dd, J=2, 8Hz), 7.83 (1H, d, J=2Hz), 7.87 (2H, d, J=8Hz)

### [Example 6] [4-[3-[1-Isopropyl-3-(4-trifluoromethylphenyl)-1H-pyrazol-5-yl]propionyl]-2-methylphenoxy]acetic acid

### (1) Ethyl [4-[3-[1-isopropyl-3-(4-trifluoromethylphenyl)-1H-pyrazol-5-yl]propionyl]-2-methylphenoxy]acetate

To a suspension of 1-(4-hydroxy-4-methylphenyl)-3-[1-isopropyl-3-(4-trifluoromethylphenyl)-1H-pyrazol-5-yl] propan-1-one (prepared in Example 5-(3), 150 mg, 0.360 mmol) and potassium carbonate (150 mg, 1.09 mmol) in acetone (10 mL) was added ethyl bromoacetate (0.12 mL, 1.08 mmol). The resulting mixture was heated under reflux for 24 hours and then cooled to room temperature. The reaction mixture was concentrated under reduced pressure. The residue was mixed with water (50 mL) and subjected to extraction with ethyl acetate (50 mL x 2). The organic portion was washed with water (30 mL) and saturated brine (30 mL) and dried over anhydrous sodium sulfate. The dried extract was placed under reduced pressure to distill the solvent off. The residue was purified by silica gel column chromatography (ethyl acetate/hexane = 1/10 to 1/5), to give the titled compound as a white crystalline product (181 mg, yield 100%).
¹H NMR (CDCl₃, 400 MHz) δ: 1.30 (3H, t, J=7Hz), 1.55 (6H, d, J=6Hz), 2.34 (3H, s), 3.09 (2H, t, J=7Hz), 3.34 (2H, t, J=7Hz), 4.27 (2H, q, J=7Hz), 4.5-4.6 (1H, m), 4.72 (2H, s), 6.36 (1H, s), 6.73 (1H, d, J=9Hz), 7.60 (2H, d, J=8Hz), 7.8-7.9 (2H, m), 7.88 (2H, d, J=8Hz)

### (2) [4-[3-[1-Isopropyl-3-(4-trifluoromethylphenyl)-1H-pyrazol-5-yl]propionyl]-2-methylphenoxy]acetic acid

The titled compound was prepared by procedures similar to the procedures of Example 5-(5).

White crystalline product
Yield 90%
mp: 160-163°C
FAB-MS (m/e): 475 (M+1)
¹H NMR (CDCl₃, 400 MHz) δ: 1.54 (6H, d, J=6Hz), 2.34 (3H, s), 3.09 (2H, t, J=7Hz), 3.33 (2H, t, J=7Hz), 4.5-4.6 (1H, m), 4.77 (2H, s), 6.35 (1H, s), 6.76 (1H, d, J=9Hz), 7.60 (2H, d, J=9Hz), 7.8-7.9 (2H, m), 7.88 (2H, d, J=8Hz)
IR (KBr, cm⁻¹): 2983, 2941, 2347, 1736, 1676, 1620, 1601, 1500, 1458, 1419, 1327, 1282, 1205, 1161, 1140, 1109, 1066, 1016, 856, 796

### [Example 7] 2-[4-[1-[2-[1-Isopropyl-3-(4-trifluoromethylphenyl)-1H-pyrazol-5-yl]ethyl]vinyl]-2-methylphenoxy]-2-methylpropionic acid

### (1) Ethyl 2-[4-[1-[2-[1-isopropyl-3-(4-trifluoromethylphenyl)-1H-pyrazol-5-yl]ethyl]vinyl]-2-methylphenoxy]-2-methyl propionate

Methyltriphenylphosphonium bromide (536 mg, 1.50 mmol) was suspended in dry tetrahydrofuran (25 mL) under a nitrogen atmosphere. To the suspension was added sodium amide (78 mg, 2.00 mmol). The mixture was stirred for 18 hours at room temperature. Subsequently, a solution of ethyl 2-[4-[3-[1-isopropyl-3-(4-trifluoromethylphenyl)-1H-pyrazol-5-yl]propionyl]-2-methylphenoxy]-2-methylpropionate (531 mg, 1.00 mmol) in dry tetrahydrofuran (10 mL) was dropwise added for 15 minutes. The resulting mixture was stirred for 30 hours at room temperature. The reaction mixture was poured into ice-water (150 mL) and subjected to extraction with ethyl acetate (150 mL x 2). The organic portion was washed with saturated brine (100 mL x 2) and dried over anhydrous sodium sulfate. The dried extract was placed under reduced pressure to distill the solvent off. The residue was purified by silica gel column chromatography (ethyl acetate/hexane = 1/20 to 1/10), to give the titled compound as a colorless oily product (95 mg, yield 18%).
¹H NMR (CDCl₃, 400 MHz) δ: 1.26 (3H, t, J=7Hz), 1.48 (6H, d, J=7Hz), 1.61 (6H, s), 2.25 (3H, s), 2.7-2.9 (4H, m), 4.25 (2H, q, J=7Hz), 4.3-4.4 (1H, m), 5.03 (1H, d, J=1Hz), 5.27 (1H, d, J=1Hz), 6.36 (1H, s), 6.64 (1H, d, J=8Hz), 7.12 (1H, dd, J=2, 8Hz), 7.23 (1H, d, J=2Hz), 7.60 (2H, d, J=8Hz), 7.89 (2H, d, J=8Hz)

### (2) 2-[4-[1-[2-[1-Isopropyl-3-(4-trifluoromethylphenyl)-1H-pyrazol-5-yl]ethyl]vinyl]-2-methylphenoxy]-2-methylpropionic acid

To a solution of the above-mentioned ethyl 2-[4-[1-[2-[1-isopropyl-3-(4-trifluoromethylphenyl)-1H-pyrazol-5-yl]ethyl]vinyl]-2-methylphenoxy]-2-methyl propionate (43 mg, 0.081 mmol) in a mixture of ethanol (4 mL) and water (2 mL) was added lithium hydroxide monohydrate (20.6 mg, 0.49 mmol). The resulting mixture was heated under reflux for 3 hours, and then cooled to room temperature. The cooled mixture was adjusted to pH 3 by adding water (20 mL) and 1M hydrochloric acid under cooling with ice. The mixture was then subjected to extraction with ethyl acetate (50 mL). The organic portion was washed with water (20 mL) and saturated brine (20 mL), and dried over anhydrous sodium sulfate. The dried extract was placed under reduced pressure to distill the solvent of, to give the titled compound as a colorless oily product (40 mg, yield 98%).
FAB-MS (m/e) : 501 (M+1)
¹H NMR (CDCl₃, 400 MHz) δ: 1.48 (6H, d, J=6Hz), 1.64 (6H, s), 2.26 (3H, s), 2.7-2.9 (4H, m), 4.25 (2H, q, J=7Hz), 4.3-4.4 (1H, m), 5.05 (1H, s), 5.28 (1H, s), 6.36 (1H, s), 6.77 (1H, d, J=8Hz), 7.14 (1H, dd, J=2, 8Hz), 7.23 (1H, d, J=2Hz), 7.60 (2H, d, J=8Hz), 7.89 (2H, d, J=8Hz)

### [Example 8] 2-[4-[3-[1-Isopropyl-3-(4-trifluoromethylphenyl)-1H-pyrazol-5-yl]-1-methylpropyl]-2-methylphenoxy]-2-methylpropionic acid

### (1) Ethyl 2-[4-[3-[1-isopropyl-3-(4-trifluoromethylphenyl)-1H-pyrazol-5-yl]-1-methylpropyl]-2-methylphenoxy]-2-methylpropionate

To a solution of ethyl 2-[4-[1-[2-[1-isopropyl-3-(4-trifluoromethylphenyl)-1H-pyrazol-5-yl]ethyl]vinyl]-2-methylphenoxy]-2-methyl propionate (obtained in Example 7-(1), 52 mg, 0.098 mmol) in ethanol (10 mL) was added 10% palladium/carbon (10 mg, 0.0093 mmol). The mixture was then subjected to catalytic hydrogenation for 24 hours at room temperature. The catalyst was filetered off, and the filtrate was concentrated under reduced pressure, to give the titled compound as a pale yellow oily product (36 mg, yield 69%).
¹H NMR (CDCl₃, 400 MHz) δ: 1.2-1.3 (6H, m), 1.44 (6H, d, J=7Hz), 1.58 (6H, s), 1.9-2.0 (2H, m), 2.24 (3H, s), 2.4-2.5 (2H, m), 2.7-2.8 (1H, m), 4.2-4.3 (3H, m), 6.30 (1H, s), 6.64 (1H, d, J=8Hz), 6.86 (1H, dd, J=2, 8Hz), 6.97 (1H, d, J=2Hz), 7.60 (2H, d, J=8Hz), 7.88 (2H, d, J=8Hz)

### (2) 2-[4-[3-[1-Isopropyl-3-(4-trifluoromethylphenyl)-1H-pyrazol-5-yl]-1-methylpropyl]-2-methylphenoxy]-2-methylpropionic acid

The titled compound was prepared by procedures similar to the procedures of Example 7-(2).
Pale yellow oily product
Yield 100%
¹H NMR (CDCl₃, 400 MHz) δ: 1.28 (3H, d, J=7Hz), 1.44 (6H, d, J=7Hz), 1.61 (6H, s), 1.9-2.0 (2H, m), 2.24 (3H, s), 2.4-2.6 (2H, m), 2.7-2.8 (1H, m), 4.2-4.3 (1H, m), 6.30 (1H, s), 6.78 (1H, d, J=8Hz), 6.91 (1H, dd, J=2, 8Hz), 7.00 (1H, d, J=2Hz), 7.59 (2H, d, J=8Hz), 7.87 (2H, d, J=8Hz)

### [Example 9] 2-[4-[3-[1-Isopropyl-3-(4-trifluoromethylphenyl)-1H-pyrazol-5-yl]propionyl]-2-methylphenoxy]propionic acid

### (1) Ethyl 2-[4-[3-[1-isopropyl-3-(4-trifluoromethylphenyl)-1H-pyrazol-5-yl]propionyl]-2-methylphenoxy]propionate

The titled compound was prepared by procedures similar to the procedures of Example 5-(4).

White crystalline product
Yield 86%
¹H NMR (CDCl₃, 400 MHz) δ: 1.25 (3H, t, J=7Hz), 1.54 (6H, d, J=7Hz), 1.67 (3H, d, J=7Hz), 2.33 (3H, s), 3.08 (2H, t, J=7Hz), 3.33 (2H, t, J=7Hz), 4.22 (2H, q, J=7Hz), 4.5-4.6 (1H, m), 4.83 (1H, q, J=7Hz), 6.36 (1H, s), 6.70 (1H, d, J=8Hz), 7.60 (2H, d, J=8Hz), 7.80 (1H, dd, J=2, 8Hz), 7.82 (1H, d, J=2Hz), 7.88 (2H, d, J=8Hz)

### (2) 2-[4-[3-[1-Isopropyl-3-(4-trifluoromethylphenyl)-1H-pyrazol-5-yl]propionyl]-2-methylphenoxy]propionic acid

The titled compound was prepared by procedures similar to the procedures of Example 7-(2).
White crystalline product
Yield 100%
FAB-MS (m/e): 489 (M+1)
¹H NMR (CDCl₃, 400 MHz) δ: 1.54 (6H, d, J=7Hz), 1. 71 (3H, d, J=7Hz), 2.32 (3H, s), 3.08 (2H, t, J=7Hz), 3.33 (2H, t, J=7Hz), 4.5-4.6 (1H, m), 4.89 (1H, q, J=7Hz), 6.35 (1H, s), 6.74 (1H, d, J=8Hz), 7.59 (2H, d, J=8Hz), 7.80 (1H, dd, J=2, 8Hz), 7.83 (1H, d, J=2Hz), 7.87 (2H, d, J=8Hz)
IR (KBr, cm⁻¹) : 3429, 2939, 2347, 1740, 1674, 1601, 1502, 1458, 1327, 1259, 1207, 1161, 1138, 1109, 1066, 970, 854, 798, 756

### [Example 10] 2-[4-[3-[1-Hexyl-3-(4-trifluoromethylphenyl)-1H-pyrazol-5-yl]propionyl]-2-methylphenoxy]-2-methylpropionic acid

### (1) Ethyl 1-hexyl-3-(4-trifluoromethylphenyl)-1H-pyrazol-5-carboxylate

The titled compound was prepared by procedures similar to the procedures of Example 5-(1).
Pale yellow oily product
Yield 88%
¹H NMR (CDCl₃, 400 MHz) δ: 0.88 (3H, t, J=7Hz), 1.2-1.4 (6H, m), 1.41 (3H, t, J=7Hz), 1.8-1.9 (2H, m), 4.38 (2H, q, J=7Hz), 4.59 (2H, t, J=7Hz), 7.17 (1H, s), 7.65 (2H, d, J=8Hz), 7.91 (2H, d, J=8Hz)

### (2) [1-Hexyl-3-(4-trifluoromethylphenyl)-1H-pyrazol-5-yl]methanol

The titled compound was prepared by procedures similar to the procedures of Example 5-(2).

White crystalline product
Yield 98%
¹H NMR (CDCl₃, 400 MHz) δ: 0.89 (3H, t, J=7Hz), 1.2-1.4 (6H, m), 1.71 (1H, t, J=6Hz), 1.9-2.0 (2H, m), 4.18 (2H, t, J=7Hz), 4.72 (2H, d, J=6Hz), 6.54 (1H, s), 7.63 (2H, d, J=8Hz), 7.88 (2H, d, J=8Hz)

### (3) 3-[1-Hexyl-3-(4-trifluoromethylphenyl)-1H-pyrazol-5-yl]-1-(4-hydroxy-3-methylphenyl)propan-1-one

To a solution of the above-mentioned [1-hexyl-3-(4-trifluoromethylphenyl)-1H-pyrazol-5-yl]methanol (1.36 g, 4.17 mmol) in a mixture of dry benzene (20 mL) and dry methylene chloride (20 mL) was added thionyl chloride (0.46 mL, 6.31 mmol) under cooling with ice. The resulting mixture was stirred for 6 hours at room temperature. The solvent was distilled off under reduced presesure, to give 5-chloromethyl-1-hexyl-3-(4-trifluoromethylphenyl)-1H-pyrazole.

Thereafter, procedures similar to the procedures of Example 5-(3) were carried out, to give the titled compound.

White crystalline product
Yield 59%
¹H NMR (CDCl₃, 400 MHz) δ: 0.86 (3H, t, J=7Hz), 1.2-1.4 (6H, m), 1.7-1.8 (2H, m), 2.17 (3H, s), 2.96 (2H, t, J=7Hz), 3.37 (2H, t, J=7Hz), 4.12 (2H, t, J=7Hz), 6.67 (1H, s), 6.86 (1H, d, J=8Hz), 7.71 (2H, d, J=8Hz), 7.75 (1H, dd, J=2, 8Hz), 7.80 (1H, d, J=2Hz), 7.93 (2H, d, J=8Hz), 10.25 (1H, s)

### (4) Ethyl 2-[4-[3-[1-hexyl-3-(4-trifluoromethylphenyl)-1H-pyrazol-5-yl]propionyl]-2-methylphenoxy]-2-methylpropionate

The titled compound was prepared by procedures similar to the procedures of Example 5-(4).

White crystalline product
Yield 89%
¹H NMR (CDCl₃, 400 MHz) δ: 0.88 (3H, t, J=7Hz), 1.22 (3H, t, J=7Hz), 1.3-1.4 (6H, m), 1.66 (6H, s), 1.8-1.9 (2H, m), 2.28 (3H, s), 3.06 (2H, t, J=7Hz), 3.33 (2H, t, J=7Hz), 4.13 (2H, t, J=7Hz), 4.23 (2H, q, J=7Hz), 6.37 (1H, s), 6.63 (1H, d, J=8Hz), 7.60 (2H, d, J=8Hz), 7.74 (1H, dd, J=2, 8Hz), 7.82 (1H, d, J=2Hz), 7.86 (2H, d, J=8Hz)

### (5) 2-[4-[3-[1-Hexyl-3-(4-trifluoromethylphenyl)-1H-pyrazol-5-yl]propionyl]-2-methylphenoxy]-2-methylpropionic acid

The titled compound was prepared by procedures similar to the procedures of Example 7-(2).

White amorphous product
Yield 86%
FAB-MS (m/e): 545 (M+1)
¹H NMR (CDCl₃, 400 MHz) δ: 0.88 (3H, t, J=7Hz), 1.2-1.4 (6H, m), 1.70 (6H, s), 1.8-1.9 (2H, m), 2.29 (3H, s), 3.06 (2H, t, J=7Hz), 3.33 (2H, t, J=7Hz), 4.13 (2H, t, J=7Hz), 6.36 (1H, s), 6.77 (1H, d, J=8Hz), 7.59 (2H, d, J=8Hz), 7.75 (1H, dd, J=2, 8Hz), 7.8-7.9 (3H, m)

### [Example 11] [4-[3-[1-Hexyl-3-(4-trifluoromethylphenyl)-1H-pyrazol-5-yl]propionyl]-2-methylphenoxy]acetic acid

### (1) Ethyl [4-[3-[1-hexyl-3-(4-trifluoromethylphenyl)-1H-pyrazol-5-yl]propionyl]-2-methylphenoxy]acetate

The titled compound was prepared by procedures similar to the procedures of Example 6-(1).

White crystalline product
Yield 100%
¹H NMR (CDCl₃, 400 MHz) δ: 0.88 (3H, t, J=7Hz), 1.30 (3H, t, J=7Hz), 1.3-1.4 (6H, m), 1.8-1.9 (2H, m), 2.34 (3H, s), 3.07 (2H, t, J=7Hz), 3.35 (2H, t, J=7Hz), 4.1307 (2H, t, J=7Hz), 4.27 (2H, q, J=7Hz), 4.72 (2H, s), 6.37 (1H, s), 6.73 (1H, d, J=9Hz), 7.61 (2H, d, J=8Hz), 7.8-7.9 (2H, m), 7.86 (2H, d, J=8Hz)

### (2) [4-[3-[1-Hexyl-3-(4-trifluoromethylphenyl)-1H-pyrazol-5-yl]propionyl]-2-methylphenoxy]acetic acid

The titled compound was prepared by procedures similar to the procedures of Example 7-(2).

White crystalline product
Yield 96%
mp: 118-123°C
FAB-MS (m/e): 517 (M+1)
¹H NMR (CDCl₃, 400 MHz) δ: 0.88 (3H, t, J=7Hz), 1.2-1.4 (6H, m), 1.8-1.9 (2H, m), 2.33 (3H, s), 3.07 (2H, t, J=7Hz), 3.34 (2H, t, J=7Hz), 4.14 (2H, t, J=7Hz), 4.77 (2H, s), 6.37 (1H, s), 6.76 (1H, d, J=9Hz), 7.60 (2H, d, J=8Hz), 7.8-7.9 (4H, m)
IR (KBr, cm⁻¹) : 2956, 2931, 2858, 1736, 1674, 1603, 1502, 1468, 1419, 1365, 1327, 1217, 1213, 1165, 1144, 1140, 1066, 1016, 989, 850, 798, 623

### [Example 12] 2-Methyl-2-[2-methyl-4-[3-[3-methyl-5-(4-trifluoromethylphenyl)thiophen-2-yl]propionyl]phenoxy]-propionic acid

### (1) 1-(4-Hydroxy-3-methylphenyl)-3-[3-methyl-5-(4-trifluoromethylphenyl)thiophen-2-yl]propan-1-one

The procedures of Example 10-(3) and Example 5-(3) were carried out using [3-methyl-5-(4-trifluoromethylphenyl)thiophen-2-yl]methanol (WO 02/092590), to give the titled compound.

Pale yellow crystalline product
Yield 85%
¹H NMR (CDCl₃, 400 MHz) δ: 2.22 (3H, s), 2.29 (3H, s), 3.1-3.3 (4H, m), 5.33 (1H, s), 6.81 (1H, d, J=8Hz), 7.10 (1H, s), 7.5-7.7 (4H, m), 7.76 (1H, dd, J=2, 8Hz), 7.80 (1H, d, J=2Hz)

### (2) Ethyl 2-methyl-2-[2-methyl-4-[3-[3-methyl-5-(4-trifluoromethylphenyl)thiophen-2-yl]propionyl]phenoxy]propionate

The titled compound was prepared by procedures similar to the procedures of Example 5-(4).

White crystalline product
Yield 92%
¹H NMR (CDCl₃, 400 MHz) δ: 1.21 (3H, t, J=7Hz), 1. 65 (6H, s), 2.22 (3H, s), 2.26 (3H, s), 3.1-3.3 (4H, m), 4.22 (2H, q, J=7Hz), 6.61 (1H, d, J=8Hz), 7.09 (1H, s), 7.57 (2H, d, J=8Hz), 7.61 (2H, d, J=8Hz), 7.71 (1H, dd, J=2, 8Hz), 7.80 (1H, d, J=2Hz)

### (3) 2-Methyl-2-[2-methyl-4-[3-[3-methyl-5-(4-trifluoromethylphenyl)thiophen-2-yl]propionyl]phenoxy]-propionic acid

The titled compound was prepared by procedures similar to the procedures of Example 5-(5).

Yellow oily product
Yield 100%
FAB-MS (m/e): 491 (M+1)
¹H NMR (CDCl₃, 400 MHz) δ: 1.69 (6H, s), 2.21 (3H, s), 2.28 (3H, s), 3.1-3.3 (4H, m), 6.76 (1H, d, J=8Hz), 7.09 (1H, s), 7.57 (2H, d, J=8Hz), 7.61 (2H, d, J=8Hz), 7.75 (1H, dd, J=2, 8Hz), 7.82 (1H, d, J=2Hz)

### [Example 13] [2-Methyl-4-[3-[3-methyl-5-(4-trifluoromethylphenyl)thiophen-2-yl]propionyl]phenoxy]acetic acid

### (1) Ethyl [2-methyl-4-[3-[3-methyl-5-(4-trifluoromethylphenyl)thiophen-2-yl]propionyl]phenoxy]acetate

The titled compound was prepared by procedures similar to the procedures of Example 6-(1).

Yellow oily product
Yield 100%
¹H NMR (CDCl₃, 400 MHz) δ: 1.29 (3H, t, J=7Hz), 2.22 (3H, s), 2.32 (3H, s), 3.1-3.3 (4H, m), 4.27 (2H, q, J=7Hz), 4.70 (2H, s), 6.71 (1H, d, J=8Hz), 7.09 (1H, s), 7.57 (2H, d, J=8Hz), 7.61 (2H, d, J=8Hz), 7.8-7.9 (2H, m)

### (2) [2-Methyl-4-[3-[3-methyl-5-(4-trifluoromethylphenyl)-thiophen-2-yl]propionyl]phenoxy]acetic acid

The titled compound was prepared by procedures similar to the procedures of Example 5-(5).

Pale yellow crystalline product
Yield 98%
FAB-MS (m/e): 463 (M+1)
¹H NMR (CDCl₃, 400 MHz) δ: 2.22 (3H, s), 2.33 (3H, s), 3.1-3.3 (4H, m), 4.77 (2H, s), 6.75 (1H, d, J=9Hz), 7.10 (1H, s), 7.57 (2H, d, J=8Hz), 7.61 (2H, d, J=8Hz), 7.8-7.9 (2H, m)

### [Example 14] 2-[4-[3-[1-Hexyl-3-(4-methylphenyl)-1H-pyrazol-5-yl]propionyl]-2-methylphenoxy]-2-methylpropionic acid

### (1) 3-(1-Hexyl-3-(4-methylphenyl)-1H-pyrazol-5-yl)-1-(4-hydroxy-3-methylphenyl)propan-1-one

The procedures of Example 5-(3) were carried out using 5-chloromethyl-1-hexyl-3-(4-methylphenyl)-1H-pyrazole and ethyl 3-(4-benzyloxy-3-methylphenyl)-3-oxopropinate, to give the titled compound.

Pale brown crystalline product
Yield 35%
¹H NMR (CDCl₃, 400 MHz) δ: 0.87 (3H, t, J=7Hz), 1.2-1.4 (6H, m), 1.8-1.9 (2H, m), 2.30 (3H, s), 2.35 (3H, s), 3.05 (2H, t, J=7Hz), 3.32 (2H, t, J=7Hz), 4.10 (2H, t, J=7Hz), 5.84 (1H, bs), 6.31 (1H, s), 6.79 (1H, d, J=8Hz), 7.17 (2H, d, J=8Hz), 7.64 (2H, d, J=8Hz), 7.74 (1H, dd, J=2, 8Hz), 7.82 (1H, d, J=2Hz)

### (2) Ethyl 2-[4-[3-[1-hexyl-3-(4-methylphenyl)-1H-pyrazol-5-yl]propionyl]-2-methylphenoxy]-2-methylpropionate

The titled compound was prepared by procedures similar to the procedures of Example 5-(4).

Colorless oily product
Yield 89%
¹H NMR (CDCl₃, 400 MHz) δ: 0.88 (3H, t, J=7Hz), 1.22 (3H, t, J=7Hz), 1.3-1.4 (6H, m), 1.66 (6H, s), 1.8-1.9 (2H, m), 2.28 (3H, s), 2.35 (3H, s), 3.04 (2H, t, J=7Hz), 3.32 (2H, t, J=7Hz), 4.10 (2H, t, J=7Hz), 4.23 (2H, q, J=7Hz), 6.29 (1H, s), 6.63 (1H, d, J=8Hz), 7.17 (2H, d, J=8Hz), 7.64 (2H, d, J=8Hz), 7.73 (1H, dd, J=2, 8Hz), 7.81 (1H, d, J=2Hz)

### (3) 2-[4-[3-[1-Hexyl-3-(4-methylphenyl)-1H-pyrazol-5-yl]propionyl]-2-methylphenoxy]-2-methylpropionic acid

The titled compound was prepared by procedures similar to the procedures of Example 7-(2).

White amorphous product
Yield 91%
FAB-MS (m/e): 491 (M+1)
¹H NMR (CDCl₃, 400 MHz) δ: 0.87 (3H, t, J=7Hz), 1.2-1.4 (6H, m), 1.70 (6H, s), 1.8-1.9 (2H, m), 2.29 (3H, s), 2.34 (3H, s), 3.04 (2H, t, J=7Hz), 3.31 (2H, t, J=7Hz), 4.09 (2H, t, J=7Hz), 6.29 (1H, s), 6.77 (1H, d, J=8Hz), 7.15 (2H, d, J=8Hz), 7.62 (2H, d, J=8Hz), 7.73 (1H, dd, J=2, 8Hz), 7.83 (1H, d, J=2Hz)

### [Example 15] 2-[4-[3-[3-Isopropyl-5-(4-trifluoromethylphenyl)thiophen-2-yl]propionyl]-2-methylphenoxy]-2-methylpropionic acid

### (1) 1-(4-Hydroxy-3-methylphenyl)-3-[3-isopropyl-5-(4-trifluoromethylphenyl)thiophen-2-yl]propan-1-one

Procedures similar to the procedures of Examples 10-(3) and 5-(3) are carried out using [3-isopropyl-5-(4-trifluoromethylphenyl)thiophen-2-yl]methanol (WO 2004/063184), to give the titled compound.

Brown crystalline product
Yield 83%
¹H NMR (CDCl₃, 400 MHz) δ: 1.25 (6H, d, J=7Hz), 2.29 (3H, s), 3.0-3.1 (1H, m), 3.2-3.3 (4H, m), 5.48 (1H, bs), 6.82 (1H, d, J=8Hz), 7.20 (1H, s), 7.58 (2H, d, J=8Hz), 7.64 (2H, d, J=8Hz), 7.76 (1H, dd, J=2, 8Hz), 7.81 (1H, d, J=2Hz)

### (2) Ethyl 2-[4-[3-[3-isopropyl-5-(4-trifluoromethylphenyl)thiophen-2-yl]propionyl]-2-methylphenoxy]-2-methylpropionate

The titled compound was prepared by procedures similar to the procedures of Example 5-(4).

Yellow oily product
Yield 91%
¹H NMR (CDCl₃, 400 MHz) δ: 1.22 (3H, t, J=7Hz), 1.25 (6H, d, J=7Hz), 1.65 (6H, s), 2.27 (3H, s), 3.0-3.1 (1H, m), 3.2-3.3 (4H, m), 4.22 (2H, q, J=7Hz), 6.62 (1H, d, J=8Hz), 7.20 (1H, s), 7.57 (2H, d, J=8Hz), 7.64 (2H, d, J=8Hz), 7.71 (1H, dd, J=2, 8Hz), 7.80 (1H, d, J=2Hz)

### (3) 2-[4-[3-[3-Isopropyl-5-(4-trifluoromethylphenyl)thiophen-2-yl]propionyl]-2-methylphenoxy]-2-methylpropionic acid

The titled compound was prepared by procedures similar to the procedures of Example 7-(2).

Pale yellow amorphous product
Yield 96%
FAB-MS (m/e): 519 (M+1)
¹H NMR (CDCl₃, 400 MHz) δ: 1.25 (6H, d, J=7Hz), 1.69 (6H, s), 2.27 (3H, s), 3.0-3.1 (1H, m), 3.2-3.3 (4H, m), 6.75 (1H, d, J=8Hz), 7.19 (1H, s), 7.57 (2H, d, J=8Hz), 7.63 (2H, d, J=8Hz), 7.75 (1H, dd, J=2, 8Hz), 7.81 (1H, d, J=2Hz)

### [Example 16] [4-[3-[3-Isopropyl-5-(4-trifluoromethylphenyl)thiophen-2-yl]propionyl]-2-methylphenoxy]acetic acid

### (1) Ethyl [4-[3-[3-isopropyl-5-(4-trifluoromethylphenyl)-thiophen-2-yl]propionyl]-2-methylphenoxy]acetate

The titled compound was prepared by procedures similar to the procedures of Example 6-(1).

Yellow oily product
Yield 97%
¹H NMR (CDCl₃, 400 MHz) δ: 1.25 (6H, d, J=7Hz), 1.29 (3H, t, J=7Hz), 2.33 (3H, s), 3.0-3.1 (1H, m), 3.2-3.3 (4H, m), 4.27 (2H, q, J=7Hz), 4.70 (2H, s), 6.72 (1H, d, J=8Hz), 7.20 (1H, s), 7.57 (2H, d, J=8Hz), 7.64 (2H, d, J=8Hz), 7.8-7.9 (2H, m)

### (2) [4-[3-[3-Isopropyl-5-(4-trifluoromethylphenyl)-thiophen-2-yl]propionyl]-2-methylphenoxy]acetic acid

The titled compound was prepared by procedures similar to the procedures of Example 7-(2).

White crystalline product
Yield 90%
mp: 139-143°C
FAB-MS (m/e): 491 (M+1)
¹H NMR (CDCl₃, 400 MHz) δ: 1.25 (6H, d, J=7Hz), 2.31 (3H, s), 3.0-3.1 (1H, m), 3.2-3.3 (4H, m), 4.74 (2H, s), 6.73 (1H, d, J=8Hz), 7.19 (1H, s), 7.57 (2H, d, J=8Hz), 7.63 (2H, d, J=8Hz), 7.8-7.9 (2H, m)
IR (KBr, cm⁻¹): 2964, 2872, 2584, 2345, 1749, 1670, 1616, 1601, 1581, 1506, 1454, 1427, 1363, 1329, 1279, 1244, 1240, 1205, 1163, 1132, 1124, 1070, 1014, 887, 829, 804, 773, 679

### [Example 17] 2-[4-[3-[3-Hexyl-5-(4-methylphenyl)thiophen-2-yl]propionyl]-2-methylphenoxy]-2-methylpropionic acid

Ethyl 2-[4-[3-[3-hexyl-5-(4-methylphenyl)thiophen-2-yl]propionyl]-2-methylphenoxy]-2-methylpropionate was prepared by procedures similar to the procedures of Example 5-(4).

Thereafter, the titled compound was prepared by procedures similar to the procedures of Example 7-(2).

Pale yellow oily product
FAB-MS (m/e): 507 (M+1)
¹H NMR (CDCl₃, 400 MHz) δ: 0.88 (3H, t, J=7Hz), 1.2-1.4 (6H, m), 1.5-1.6 (2H, m), 1.69 (6H, s), 2.28 (3H, s), 2.34 (3H, s), 2.53 (2H, t, J=7Hz), 3.1-3.3 (4H, m), 6.77 (1H, d, J=8Hz), 6.99 (1H, s), 7.14 (2H, d, J=8Hz), 7.43 (2H, d, J=8Hz), 7.75 (1H, dd, J=2, 8Hz), 7.82 (1H, d, J=2Hz)

### [Example 18] 4-[3-[1-Cyclopropylmethyl-3-(4-trifluoromethylphenyl)-1H-pyrazol-5-yl]propionyl]-2-methylphenoxyacetic acid

### (1) 3-[1-Cyclopropylmethyl-3-(4-trifluoromethylphenyl)-1H-pyrazol-5-yl]-1-(4-hydroxy-3-methylphenyl)propan-1-one

The procedures of Example 5-(3) were carried out using ethyl 5-chloromethyl-1-cyclopropylmethyl-3-(4-trifluoromethylphenyl)-1H-pyrazole and ethyl 3-(4-benzyloxy-3-methylphenyl)-3-oxopropionate, to give the titled compound.

White crystalline product
Yield 38%
¹H NMR (CDCl₃, 400 MHz) δ: 0.4-0.7 (4H, m), 1.3-1.4 (1H, m), 2.31 (3H, s), 3.10 (2H, t, J=7Hz), 3.35 (2H, t, J=7Hz), 4.07 (2H, t, J=7Hz), 5.25 (1H, s), 6.40 (1H, s), 6.82 (1H, d, J=8Hz), 7.61 (2H, d, J=8Hz), 7.78 (1H, dd, J=2, 8Hz), 7.82 (1H, d, J=2Hz), 7.87 (2H, d, J=8Hz)

### (2) Ethyl 4-[3-[1-cyclopropylmethyl-3-(4-trifluoromethylphenyl)-1H-pyrazol-5-yl]propionyl]-2-methylphenoxyacetate

The titled compound was prepared by procedures similar to the procedures of Example 5-(4).

White crystalline product
Yield 80%

### (3) 4-[3-[1-Cyclopropylmethyl-3-(4-trifluoromethylphenyl)-1H-pyrazol-5-yl]propionyl]-2-methylphenoxyacetic acid

The titled compound was prepared by procedures similar to the procedures of Example 7-(2).

White crystalline product
mp: 168-175°C
Yield 91%
¹H NMR (CDCl₃, 400 MHz) δ: 0.4-0.7 (4H, m), 1.3-1.4 (1H, m), 2.34 (3H, s), 3.10 (2H, t, J=7Hz), 3.35 (2H, t, J=7Hz), 4.07 (2H, d, J=7Hz), 4.78 (2H, s), 6.40 (1H, s), 6.77 (1H, d, J=8Hz), 7.60 (2H, d, J=8Hz), 7.8-8.0 (4H, m)

### [Example 19] 2-[4-[3-[1-Cyclopropylmethyl-3-(4-trifluoromethylphenyl)-1H-pyrazol-5-yl]propionyl]-2-methylphenoxy]-2-methylpropionic acid

### (1) Ethyl 2-[4-[3-[1-cyclopropylmethyl-3-(4-trifluoromethylphenyl)-1H-pyrazol-5-yl]propionyl]-2-methylphenoxy]-2-methylpropionate

The titled compound was prepared by procedures similar to the procedures of Example 5-(4).

Colorless oily product
Yield 59%
¹H NMR (CDCl₃, 400 MHz) δ: 0.4-0.7 (4H, m), 1.22 (3H, t, J=7Hz), 1.3-1.4 (1H, m), 1.66 (6H, s), 2.28 (3H, s), 3.10 (2H, t, J=7Hz), 3.34 (2H, t, J=7Hz), 4.06 (2H, d, J=7Hz), 4.23 (2H, q, J=7Hz), 6.40 (1H, s), 6.64 (1H, d, J=8Hz), 7.60 (2H, d, J=8Hz), 7.73 (1H, dd, J=2, 8Hz), 7.82 (1H, d, J=2Hz), 7.87 (2H, d, J=8Hz)

### (2) 2-[4-[3-[1-Cyclopropylmethyl-3-(4-trifluoromethylphenyl)-1H-pyrazol-5-yl]propionyl]-2-methylphenoxy]-2-methylpropionic acid

The titled compound was prepared by procedures similar to the procedures of Example 7-(2).

White crystalline product
mp: 55-60°C
¹H NMR (CDCl₃, 400 MHz) δ: 0.4-0.7 (4H, m), 1.3-1.4 (1H, m), 1.70 (6H, s), 2.29 (3H, s), 3.10 (2H, t, J=7Hz), 3.34 (2H, t, J=7Hz), 4.08 (2H, d, J=7Hz), 6.40 (1H, s), 6.77 (1H, d, J=8Hz), 7.60 (2H, d, J=8Hz), 7.75 (1H, dd, J=2, 8Hz), 7.83 (1H, d, J=2Hz), 7.86 (2H, d, J=8Hz)

### [Example 20] 2-[4-[3-[1-Benzyl-3-(4-trifluoromethylphenyl)-1H-pyrazol-5-yl]propionyl]-2-methylphenoxy]-2-methylpropionic acid

### (1) 3-[1-Benzyl-3-(4-trifluoromethylphenyl)-1H-pyrazol-5-yl]-1-(4-hydroxy-3-methyphenyl)propan-1-one

The procedures of Example 5-(3) were carried out using 5-chloromethyl-1-benzyl-3-(4-trifluoromethylphenyl)-1H-pyrazole and ethyl 3-(4-benzyloxy-3-methylphenyl)-3-oxopropionate, to give the titled compound.

White crystalline product
Yield 50%
¹H NMR (CDCl₃, 400 MHz) δ: 2.27 (3H, s), 3.00 (2H, t, J=7Hz), 3.13 (2H, t, J=7Hz), 5.44 (3H, s), 6.46 (1H, s), 6.76 (1H, d, J=8Hz), 7.1-7.4 (5H, m), 7.6-7.8 (4H, m), 7.90 (2H, d, J=8Hz)

### (2) Ethyl 2-[4-[3-[1-benzyl-3-(4-trifluoromethylphenyl)-1H-pyrazol-5-yl]propionyl]-2-methylphenoxy]-2-methylpropionate

The titled compound was prepared by procedures similar to the procedures of Example 5-(4).

White crystalline product
Yield 65%
¹H NMR (CDCl₃, 400 MHz) δ: 1.21 (3H, t, J=7Hz), 1.65 (6H, s), 2.25 (3H, s), 2.99 (2H, t, J=7Hz), 3.13 (2H, t, J=7Hz), 4.22 (2H, q, J=7Hz), 5.43 (2H, s), 6.45 (1H, s), 6.59 (1H, d, J=8Hz), 7.1-7.4 (5H, m), 7.60 (1H, dd, J=2, 8Hz), 7.63 (1H, d, J=8Hz), 7.70 (1H, d, J=2Hz), 7.90 (2H, d, J=8Hz)

### (3) 2-[4-[3-[1-Benzyl-3-(4-trifluoromethylphenyl)-1H-pyrazol-5-yl]propionyl]-2-methylphenoxy]-2-methylpropionic acid

The titled compound was prepared by procedures similar to the procedures of Example 7-(2).

White crystalline product
mp: 55°C
¹H NMR (CDCl₃, 400 MHz) δ: 1.68 (6H, s), 2.26 (3H, s), 3.00 (2H, t, J=7Hz), 3.13 (2H, t, J=7Hz), 5.44 (2H, s), 6.45 (1H, s), 6.73 (1H, d, J=8Hz), 7.1-7.4 (5H, m), 7.62 (1H, d, J=8Hz), 7.62 (1H, dd, J=2Hz, 8Hz), 7.71 (1H, d, J=2Hz), 7.89 (2H, d, J=8Hz)

### [Example 21] 4-[3-[1-Benzyl-3-(4-trifluoromethylphenyl)-1H-pyrazol-5-yl]propionyl]-2-methylphenoxyacetic acid

### (1) Ethyl 4-[3-[1-benzyl-3-(4-trifluoromethylphenyl)-1H-pyrazol-5-yl]propionyl]-2-methylphenoxyacetate

The titled compound was prepared by procedures similar to the procedures of Example 5-(4).

White crystalline product
Yield 96%
¹H NMR (CDCl₃, 400 MHz) δ: 1.29 (3H, t, J=7Hz), 2.31 (3H, s), 3.00 (2H, t, J=7Hz), 3.14 (2H, t, J=7Hz), 4.27 (2H, q, J=7Hz), 4.70 (2H, s), 5.44 (2H, s), 6.45 (1H, s), 6.69 (1H, d, J=8Hz), 7.1-7.4 4 (5H, m), 7.62 (2H, d, J=8Hz), 7.7-7.8 (2H, m), 7.91 (2H, d, J=8Hz)

### (2) 4-[3-[1-Benzyl-3-(4-trifluoromethylphenyl)-1H-pyrazol-5-yl]propionyl]-2-methylphenoxyacetic acid

The titled compound was prepared by procedures similar to the procedures of Example 7-(2).

White crystalline product
mp: 175-180°C
¹H NMR (CDCl₃, 400 MHz) δ: 2.32 (3H, s), 3.00 (2H, t, J=7Hz), 3.13 (2H, t, J=7Hz), 4.76 (2H, s), 5.45 (2H, s), 6.45 (1H, s), 6.71 (1H, d, J=8Hz), 7.1-7.4 (5H, m), 7.62 (2H, d, J=8Hz), 7.7-7.8 (2H, m), 7.89 (2H, d, J=8Hz)

### [Example 22] Pharmacological Experimental

### I. Procedures of Experimental

The PPARδ activating effects of test compounds (compounds of Examples) were measured by the following method:
A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL) (Kliewer, S.A., et. al.,(1992) Nature, 358:771-774) are transfected into CV-1 cells (ATCC). After gene transfer utilizing a lipofection reagent DMRIE-C or Lipofectamin 2000 (Invitrogen), it is incubated for approx. 40 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions: a relative activity of PPAR α is calculated in consideration of a luciferase activity (assigned to 100%) of cells treated with the compound described in Example 2 of the aforementioned Patent Publication 4 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516.

### II. Experimental Results of

Experimental Results are set forth in Table 27.

**Table 27**

| | PPAR activity (%) | | |
|---|---|---|---|
| Test compound | α | γ | δ |
| Example 1 | 62 | 7 | 87 |
| Example 2 | IA | IA | 76 |
| Example 3 | 131 | 30 | 72 |
| Example 5 | 109 | 15 | 93 |
| Example 6 | IA | IA | 86 |
| Example 7 | 84 | 38 | 72 |
| Example 8 | 89 | 61 | 45 |
| Example 9 | 73 | 29 | 59 |
| Example 10 | 77 | 54 | 53 |
| Example 11 | IA | IA | 81 |
| Example 12 | 51 | 29 | 94 |
| Example 13 | IA | IA | 82 |

| | | | |
|---|---|---|---|
| PPAR activity: relative value (%) of the test compound (10-⁶M) to 100% of the control compound α: Compound described in Example 2 of Patent Publication 4 - 10⁻⁶ M γ: Rosiglitazone - 10⁻⁵ M δ: GW-501516 - 10⁻⁷ M IA: inactive Test compounds of Examples 8 and 10 for α: 10⁻⁷ M Test compounds of Examples 5, 8 and 10 for δ: 10⁻⁷ M | | | |

As is clear from Table 27, the compounds of Examples show excellent PPAR δ-activating effect.

### [Example 23] Pharmacological Experimental

The experimental results Table 28 were obtained by procedures similar to the procedures for pharmacological experimental described in Example 22.

**Table 28**

| | PPAR activity (%) | | |
|---|---|---|---|
| Test compound | α | γ | δ |
| Example 14 | 123 | 48 | 94 |
| Example 15 | 99 | 11 | 85 |
| Example 16 | inactive | inactive | 92 |
| Example 17 | 109 | 35 | 76 |

| | | | |
|---|---|---|---|
| PPAR activity: relative value (%) of the test compound (10⁻⁶M) to 100% of the control compound α: Compound described in Example 2 of Patent Publication 4 - 10⁻⁶ M γ: Rosiglitazone - 10⁻⁵ M δ: GW-501516 - 10⁻⁷ M | | | |

As is clear from Table 28, the compounds of Examples show excellent PPAR δ-activating effect.

## Claims

1. A compound having the following formula (I) or a salt thereof: 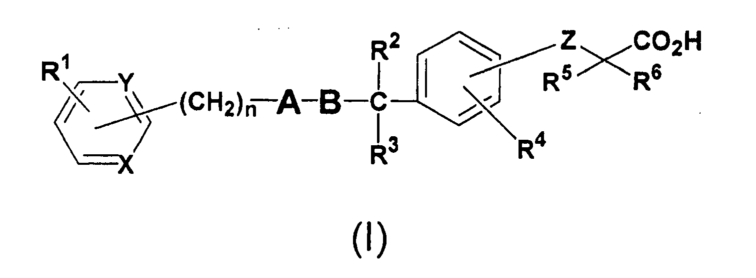 in which
R¹ and R⁴ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;
R² represents a hydrogen atom;
R³ represents an alkyl group having 1 to 8 carbon atoms, or R³ is combined with R² to represent =O or =C(R⁷)(R⁸) in which R⁷ and R⁸ are the same or different and each represents a hydrogen atom or an alkyl group having 1 to 8 carbon atoms;
R⁵ and R⁶ are the same or different and each represents a hydrogen atom, an alkyl group having 1 to 8 carbon atoms, or an alkyl group having 1 to 8 carbon atoms and a halogen atom substituent;
X and Y are the same or different and each represents CH or N;
Z represents an oxygen atom or a sulfur atom;
A represents a 5-membered heterocyclic group selected from the group consisting of pyrazole, thiophene, furan and pyrrole which optionally has an alkyl substituent having 1 to 8 carbon atoms which has a substituent selected from the group consisting of an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group which has 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and 5- or 6-membered heterocyclic group;
B represents an alkylene chain having 1 to 8 carbon atoms which optionally has a substituent selected from the group consisting of an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, and an alkoxy group having 1 to 8 carbon atoms and a halogen atom substituent, the alkylene group optionally having a double bond in the case that the alkylene group has 2 to 6 carbon atoms;
and
n is an integer of 0 to 5.

2. The compound or a salt thereof according to claim 1, in which A is pyrazole.

3. The compound or a salt thereof according to claim 2, in which -(CH₂)ₙ- is attached the pyrazole at 1-position thereof.

4. The compound or a salt thereof according to claim 2, in which -(CH₂)ₙ- is attached the pyrazole at 3-position thereof.

5. The compound or a salt thereof according to claim 3 or 4, in which -B- is attached the pyrazole at 4-or 5-position thereof.

6. The compound or a salt thereof according to claim 1, in which A is thiophene, furan or pyrrole.

7. The compound or a salt thereof according to claim 6, in which -(CH₂)ₙ- is attached the 5-membered heterocyclic group at 2-position thereof.

8. The compound or a salt thereof according to claim 1, in which A is thiophene.

9. The compound or a salt thereof according to claim 8, in which -(CH₂)ₙ- is attached the thiophene at 2-position thereof.

10. The compound or a salt thereof according to any one of claims 1 to 9, in which n is 0.

11. The compound or a salt thereof according to any one of claims 1 to 10, in which each of X and Y is CH.

12. The compound or a salt thereof according to any one of claims 1 to 11, in which R² is combined with R³ to represent =O.

13. The compound or a salt thereof according to any one of claims 1 to 12, in which B represents an alkylene. chain having 2 to 4 carbon atoms which optionally has a substituent selected from the group consisting of an alkyl group having 1 to 8 carbon atoms and an alkyl group having 1 to 8 carbon atoms and a halogen atom substituent.

14. The compound or a salt thereof according to any one of claims 1 to 12, in which B is an ethylene chain.

15. The compound or a salt thereof according to any one of claims 1 to 14, in which R¹ and R⁴ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent.

16. The compound or a salt thereof according to any one of claims 1 to 15, in which R⁵ and R⁶ are the same or different and each represents a hydrogen atom or an alkyl group having 1 to 8 carbon atoms.

17. The compound or a salt thereof according to any one of claims 1 to 16, in which the substituent optionally attached to the heterocyclic group for A is an alkyl group having 1 to 8 carbon atoms or an alkyl group having 1 to 8 carbon atom and a halogen atom substituent.

18. A compound having the following formula (II) or a salt thereof: 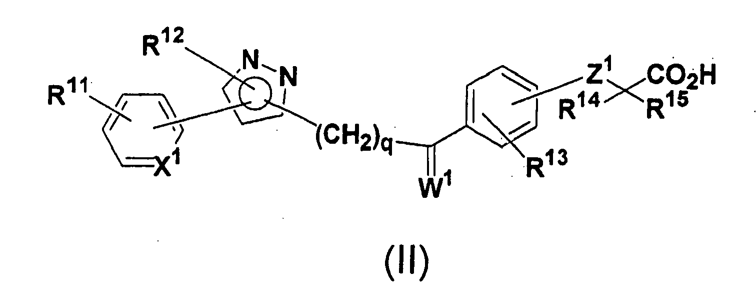 in which
R¹¹ and R¹³ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;
R¹² represents a hydrogen atom, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent;
R¹⁴ and R¹⁵ are the same or different and each represents a hydrogen atom, an alkyl group having 1 to 8 carbon atoms, or an alkyl group having 1 to 8 carbon atoms and a halogen atom substituent;
X¹ represents CH or N;
Z¹ represents an oxygen atom or a sulfur atom;
W¹ represents an oxygen atom or CH₂;
and
q is an integer of 2 to 4.

19. The compound or a salt thereof according to claim 18, in which X¹ is CH.

20. The compound or a salt thereof according to claim 18, in which R¹¹-phenyl or R¹¹-pyridyl is attached to the pyrazole at 1-position.

21. The compound or a salt thereof according to claim 18, in which R¹¹-phenyl or R¹¹-pyridyl is attached to the pyrazole at 3-position.

22. The compound or a salt thereof according to any one of claims 18 to 21, in which -(CH₂)_{q}C(=W¹) - is attached to the pyrazole at 4- or 5-position.

23. The compound or a salt thereof according to any one of claims 18 to 22, in which W¹ is an oxygen atom.

24. The compound or a salt thereof according to any one of claims 18 to 23, in which R¹¹ and R¹³ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent.

25. The compound or a salt thereof according to any one of claims 18 to 23, in which R¹¹ and R¹³ are the same or different and each represents an alkyl group having 1 to 8 carbon atoms or an alkyl group having 1 to 8 carbon atoms and a halogen atom substituent.

26. The compound or a salt thereof according to any one of claims 18 to 25, in which R¹⁴ and R¹⁵ are the same or different and each represents a hydrogen atom or an alkyl group having 1 to 8 carbon atoms.

27. The compound or a salt thereof according to any one of claims 18 to 26, in which R¹² represents a hydrogen atom, an alkyl group having 1 to 8 carbon atoms, an alkoxy group having 1 to 8 carbon atoms, an alkyl group having 1 to 8 carbon atoms and a halogen atom substituent, or an alkoxy group having 1 to 8 carbon atoms and a halogen atom substituent.

28. The compound or a salt thereof according to any one of claims 18 to 26, in which R¹² represents an alkyl group having 1 to 8 carbon atoms, or an alkyl group having 1 to 8 carbon atoms and a halogen atom substituent.

29. The compound or a salt thereof according to any one of claims 18 to 28, in which q is 2.

30. A compound having the following formula (III) or a salt thereof: 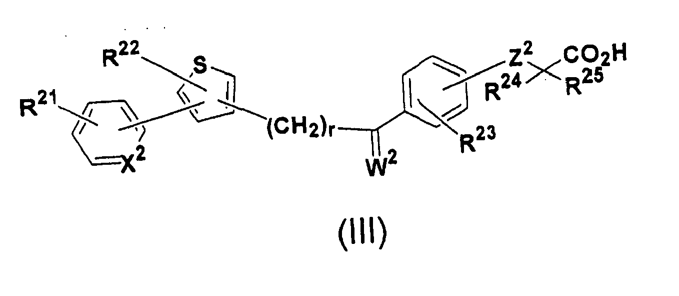 in which
R²¹ and R²³ are the same or different and each represents a hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and a halogen atom substituent, an alkoxy group having 1 to 8 carbon atoms and a halogen atom substituent, a hydroxyl group, a nitro group, an acyl group having 2 to 8 carbon atoms, an aryl group having 6 to 10 carbon atoms, or a 5- or 6-membered heterocyclic group;
R²² represents a hydrogen atom, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl group substituent, an alkyl group having 1 to 8 carbon atoms and a halogen atom substituent, an alkoxy group having 1 to 8 carbon atoms and a halogen atom substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent;
R²⁴ and R²⁵ are the same or different and each represents a hydrogen atom, an alkyl group having 1 to 8 carbon atoms, or an alkyl group having 1 to 8 carbon atoms and a halogen atom substituent;
X² represents CH or N;
Z² represents an oxygen atom or a sulfur atom;
w² represents an oxygen atom or CH₂;
and
r is an integer of 2 to 4.

31. The compound or a salt thereof according to claim 30, in which X² is CH.

32. The compound or a salt thereof according to claim 30, in which R²¹-phenyl or R²¹-pyridyl is attached to the thiophene at 2-position.

33. The compound or a salt thereof according to any one of claims 30 to 32, in which W² is an oxygen atom.

34. The compound or a salt thereof according to any one of claims 30 to 33, in which R²¹ and R²³ are the same or different and each represents a hydrogen atom, an alkyl group having 1 to 8 carbon atoms, an alkoxy group having 1 to 8 carbon atoms, a halogen atom, an alkyl group having 1 to 8 carbon atoms and a halogen atom substituent, or an alkoxy group having 1 to 8 carbon atoms and a halogen atom substituent.

35. The compound or a salt thereof according to any one of claims 30 to 33, in which R²¹ and R²³ are the same or different and each represents an alkyl group having 1 to 8 carbon atoms or an alkyl group having 1 to 8 carbon atoms and a halogen atom substituent.

36. The compound or a salt thereof according to any one of claims 30 to 35, in which R²⁴ and R²⁵ are the same or different and each represents a hydrogen atom or an alkyl group having 1 to 8 carbon atoms.

37. The compound or a salt thereof according to any one of claims 30 to 36, in which R²² represents a hydrogen atom, an alkyl group having 1 to 8 carbon atoms, an alkoxy group having 1 to 8 carbon atoms, an alkyl group having 1 to 8 carbon atoms and a halogen atom substituent, or an alkoxy group having 1 to 8 carbon atoms and a halogen atom substituent.

38. The compound or a salt thereof according to any one of claims 30 to 36, in which R²² represents an alkyl group having 1 to 8 carbon atoms, or an alkyl group having 1 to 8 carbon atoms and a halogen atom substituent.

39. The compound or a salt thereof according to any one of claims 30 to 38, in which r is 2.

40. An activator for peroxisome proliferator activated receptor δ containing a compound or a salt thereof according to any one of claims 1 to 39 as an effective component.
